# EUROPEAN PATENT SPECIFICATION

(11) **EP 2 841 402 B1**
(45) Date of publication and mention of the grant of the patent: **30.11.2016**
(21) Application number: 13725814.1
(22) Date of filing: 19.04.2013
(51) Int. Cl.: C07B 59/00, C07D 241/44, C07D 403/12, C07F 7/22

(54) **LABELLED QUINOXALINE DERIVATIVES AS MULTIMODAL RADIOPHARMACEUTICALS AND THEIR PRECURSORS**
MARKIERTE CHINOXALIN-DERIVATE ALS MULTIMODALE RADIOPHARMAZEUTIKA UND IHRE VORLÄUFER
DÉRIVÉS DE QUINOXALINE MARQUÉS COMME PRODUITS RADIOPHARMACEUTIQUES MULTIMODAUX ET LEURS PRÉCURSEURS

(30) Priority: 24.04.2012 EP 12165413
(43) Date of publication of application: 04.03.2015
(73) Proprietor: Institut National de la Santé et de la Recherche Médicale, 75013 Paris (FR); Laboratoires Cyclopharma, 63360 Saint Beauzire (FR); Universite d'Auvergne Clermont I, 63000 Clermont Ferrand (FR)
(72) Inventor: CHEZAL, Jean-Michel, F-63000 Clermont-Ferrand (FR); RBAH-VIDAL, Latifa, 44007 Nantes (FR); BILLAUD, Emilie, 17000 La Rochelle (FR); AUZELOUX, Philippe, F-63000 Clermont-Ferrand (FR); MADELMONT, Jean-Claude, F-63000 Clermont-Ferrand (FR); VIDAL, Aurélien, F-44817 Saint-Herblain Cedex (FR); MIOT-NOIRAULT, Elisabeth, F-63000 Clermont-Ferrand (FR); PAPON, Janine, F-63370 Lempdes (FR); MAISONIAL, Aurélie, F-63000 Clermont-Ferrand (FR)
(74) Representative: Lavoix
(86) International application number: PCT/IB2013/053106
(87) International publication number: WO 2013/160808

(56) References cited:
- EP-A1- 2 085 390

## Description

The present invention relates to new aromatic and heteroaromatic analogues of halobenzamides, and namely quinoxaline derivatives, which once labelled with suitable radioactive isotopes are usable as radiopharmaceuticals for the diagnosis by single photon emission computed tomography (SPECT) or positron emission tomography (PET) or for the targeted radionuclide therapy of melanoma. The present invention also relates to their synthesis process and also to their corresponding use and methods in imaging and radiotherapy.

Melanoma is one of the most dangerous skin tumours with a steadily increasing incidence. This is a highly invasive cancer, the development of which is rapidly fatal at the metastatic stage. 5-year survival does not exceed 14%, except in the case where the thickness of the tumour is less than 0.76 mm. In the case where the lesion exceeds this thickness, this tumour gives metastases in an unpredictable and silent fashion. This is why a search is currently underway for a method of investigation which makes possible the early evaluation both of the local extension and of the distant extension of the tumour. Moreover during the last decade, a number of radiopharmaceutical products, selected for their potential melanin affinity, have been experimented but few have had a satisfactory clinical development.

Early detection is crucial for prognosis. The only method now valuable for staging primary melanoma remains the sentinel lymph node biopsy, which is much more sensitive. The diagnostic and clinical utility of [¹⁸F]FDG PET/CT imaging is best for patients with more advanced stages of disease.

Several biochemical targeting systems incorporating a lot of radionuclides for diagnosis have been also evaluated for early detection in SPECT or PET imaging of melanoma metastases, as example [¹²³I]methylene blue dye, α-MSH analogues radio labelled with fluorine-18, technetium-99m, indium-111, yttrium-86 or copper-64, σ1 radioligands such as [¹⁸F]-1-(3-fluoropropyl)-4-(4-cyanophenoxymethyl)piperidine, analogues of thymidine or DOPA and various radiolabelled iodobenzamides.

If melanoma is diagnosed early (stage I or II), the most efficient treatment remains surgery. The malignant localized lesions, wherein the tumour thickness is <1.5 mm, can be cured by total resection. In this case, the success rates are approaching 90% survival at 5 years. Unfortunately, for tumours discovered when the skin lesion is already thick or ulcerated, so in case of an increased risk of metastatic disease (stage III or IV), the prognosis is very poor. In fact, the median survival rate is around 6 months and the success rates only reach 5% survival at 5 years, due to the lack of efficient therapies for metastatic malignant melanoma. For the postoperative adjuvant therapies, beneficial results were reported only for Interferon-α, which is the most commonly used to decrease the risk of recurrence. However, high doses usually administred cause substantial toxicity. The United States Food and Drug Administration have approved only three agents for the treatment of stage IV melanoma: dacarbazine, interleukin-2 and ipilimumab. Dacarbazine is an alkylating agent often used for chemotherapy. The overall response rate with this single therapy is around 22% and the median response duration is from 4 to 6 months. The oral analogue of dacarbazine, temozolomide, has also been developed. The response rate with this cytotoxic alkylating agent is comparable to this previously obtained. Interleukin-2 is a recombinant hormone of the immune system originally described as a T-cell-derivated growth factor. The response rate obtained with this therapy remains low (16%) and the treatment is often associated with significant toxic effects due to the high doses administred. More recently, a fully human antibody (ipilimumab) binding to CTLA-4, used alone or in combination with vaccines, IL2 or chemotherapy, demonstrated similar overall response rate ranges from 13 to 22%. But, none of these drugs has been shown to significantly prolong the survival of stage IV patients. Moreover, external beam radiotherapy cannot be considered as an alternative because melanoma is described as radioresistant. Several clinical trials are under way in order to find novel efficient treatments for metastatic melanoma, in the fields of chemo, immuno or radiotherapy, and combined therapies (as biochemotherapy for example). At this time, no real benefit in term of global survival has been demonstrated.

It should be explained that radiopharmaceutical products comprise two functional components, one being radioactive and the other not being radioactive. The radioactive component makes possible the detection of the product in the context of the diagnosis and it constitutes the active agent in the case of therapeutic use. It is a radionuclide with appropriate physical properties. The nonradioactive component, for its part, is a molecule or tracer, optionally biological, intended to accumulate in the target organ, in the context of the present invention, in the melanoma, and to ensure the absorption of radioactivity by the latter. This nonradioactive component is determining for the biological behaviour of the radiopharmaceutical product in the body, in particular regarding the specificity and the pharmacokinetic profile.

Document EP 458 886 describes compounds of use in the diagnosis and treatment of malignant melanoma. From these molecules, *N*-(2-diethylaminoethyl)-4-iodobenzamide (BZA) forms in particular the subject of more detailed studies, and also *N*-(2-diethylaminoethyl)-2-iodobenzamide (BZA2), in the medical imaging application and more particularly for the scintigraphic detection of primary ocular melanoma and metastases of cutaneous and ocular melanomas. BZA2, radiolabelled with iodine-123, is object of a clinical trial for melanoma metastases imaging by SPECT.

The document US 5,911,970 for its part describes, *inter alia,* other benzamide-derived compounds exhibiting a high specificity and affinity to cell surface sigma receptors of cancer cells.

Document WO 2005/089815 describes some radiohalogenated benzamide derivatives and their potential use for tumour diagnosis and tumour therapy of melanoma.

Document WO2009/095872 discloses aromatic and heteroaromatic analogues of halobenzamides in the same application.

There thus exists, on one hand, a need to have available a specific tracer which makes it possible, from the time of the diagnosis, to carry out an assessment of extension of the disease and then, subsequently, monitoring and dosimetry studies. Such a tracer should advantageously make it possible the differential diagnosis of ocular melanoma, primary lesion, which is often difficult to identify.

On the other hand, as exposed above, treatments remain defeated with regard to disseminated melanoma and the development of novel specific therapeutic approaches for the treatment of melanoma is essential.

There is moreover a need to find new molecules, which depending on the radioisotope which is introduced thereon, are able to form a radiotracer usable for the diagnosis using PET or SPECT imaging and able to form an agent for the targeted radionuclide therapy.

There is lastly a need for multimodal radiotracers widening the scope of imaging techniques in the field of diagnosis of melanoma, and more particularly a need of new tracers finding potential application in fluorine-18 PET imaging.

Today, fluorine-18, beyond its adequate physical and nuclear characteristics, appears as the most attractive positron-emitting radioisotope for radiopharmaceutical chemistry and PET imaging, part of this continuous growing interest probably due to the successful use in clinical oncology of [¹⁸F]FDG, the most widely used PET-radiopharmaceutical. Briefly, fluorine-18 displays simple decay and emission properties with a high 97% positron abundance. Compared with the other conventional short-lived positron-emitting radionuclides carbon-11, nitrogen-13 and oxygen-15, fluorine-18 has a relatively low positron energy (maximum 635 keV) and the shortest positron linear range in tissue (2.3 mm), resulting in the highest resolution in PET imaging. Its half-life (109.8 min) is long enough to give access to relatively extended imaging protocols compared to what is possible with carbon-11, therefore facilitating kinetic studies and high-quality plasma metabolite analysis. Moreover, from a chemical point of view, fluorine-18 allows multi-step synthetic approaches that can be extended over hours. Finally, fluorine-18 can be reliably and routinely produced at the multi-Curie level, using the well-characterized (p, n) nuclear reaction on an oxygen-18-enriched water target on widely implemented biomedical cyclotrons of a relatively low-energy proton beam (*e.g.,* 18 MeV).

To conclude, the use of a specific tracer, radio labelled by fluorine-18, in PET imaging could lead to an increase of sensitivity of this technique, compared with [¹⁸F]FDG, especially for the diagnosis of stage I (ocular) or II melanoma and the differential diagnosis with other tumours. Use of PET imaging is complementary to SPECT imaging and even in some contexts PET imaging, and more particularly fluorine-18 PET imaging, compared to SPECT allows better performances in terms of image quality or quantification of uptake namely for dosimetry.

Thus, the object of the present invention is the targeting of melanoma lesions by the development of molecules which, administered to the body, will make it possible to vectorize a radioisotope. The present invention therefore concerns two fields of applications, namely imaging and targeted radionuclide therapy, depending on the nature of the labelling.

Moreover the compounds in accordance with the present invention are of advantage both for their use in imaging, namely for the diagnosis of malignant melanoma, and in targeted radionuclide therapy, targeting more particularly secondary lesions and primary ocular lesions. One of the major advantages of the compounds according to the present invention lies precisely in their mixed potentiality or bimodality property. In other words, according to the chemical variations under consideration, their respective behaviours in the body destine them more particularly for use in medical imaging, for use in targeted radionuclide therapy or else, and these compounds may prove to be particularly attractive when they are able to be used both in medical imaging and in radionuclide therapy. Said molecules endowed with said bimodal potentiality are of particular interest.

To this end, the compounds of the present invention all comprise at least a fluorine atom so that they are more particularly dedicated to reach an application in PET imaging when said at least one fluorine atom is labelled into fluorine-18 but also comprise another radionuclide and/or halogen allowing them to reach another imaging potentiality of melanoma (for example SPECT) and/or a therapeutic property against melanoma.

Finally, some compounds corresponding to the general formula of the compounds in accordance with the present invention, when they are unlabelled, may also exhibit an intrinsic antitumour activity. Thus, in this specific case, one can say that the nonradioactive component defined above can play, in addition to its role of targeting the melanoma lesions and/or of promoting absorption of radioactivity, a role of cytotoxic agent *per se.* However, even if some compounds of the present invention may exhibit a chemotherapy potentiality, it is clearly the activity *via* labelling by a radioactive isotope which is targeted in the present invention.

Moreover some of the compounds encompassed within the scope of formula (I) are precursors of the radiolabelled compounds.

According to a first aspect, a subject-matter of the present invention relates to a compound of formula (I): 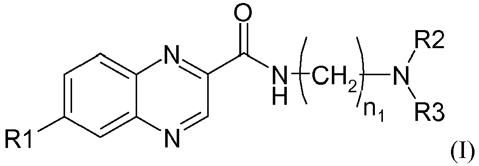 in which
R1 represents Sn(R)₃, B(OH)₂, B(OR)₂, a halogen atom, NO₂, a radionuclide or a -N⁺(R)₃ group, where R is a (C₁-C₆) alkyl group,
n₁ is an integer varying from 1 to 4,
R₂ represents a hydrogen atom or a (C₁-C₆)alkyl group,
R3 represents:
   - a -(CH₂CH₂O)ₙ₂-(CH₂)ₙ₃-X group, where n₂ is an integer varying from 1 to 12 and n₃ is an integer varying from 1 to 4, or
   - a -(CH₂CH₂O)ₙ₄-(CH₂)ₙ₅-Y group with n₄ representing an integer varying from 0 to 12, n₅ representing an integer from 1 to 4, and Y representing a -C=C-H, a -N₃ or a -Ar-(CH₂)ₙ₆-(OCH₂CH₂)ₙ₇-X group with
Ar being 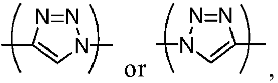 n₆ representing an integer varying from 1 to 4 and n₇ an integer varying from 0 to 12, and
X represents a halogen atom, a radionuclide or a-OSO₂R' group, where R' is a CF₃, CH, *t*-Bu, Ph, *p*-NO₂Ph, *p*-BrPh or *p*-CH₃Ph group,
and their addition salts with pharmaceutically acceptable acids.

The following groups: a halogen with the exclusion of fluorine, or a-OSO₂R' group, wherein R' is a CF₃, CH₃, *t*-Bu, Ph, *p*-NO₂Ph, *p*-BrPh or *p*-CH₃Ph group are precursor groups of an optionally labelled fluorine atom.

The following groups: a halogen, Sn(R)₃, B(OH)₂, B(OR)₂ where R is a (C₁-C₆) alkyl group are precursor group of an optionally labelled iodine atom.

According to a particular embodiment, the present invention is directed to a compound of formula (I) as defined above, wherein R2 is an ethyl group.

According to another particular embodiment, the present invention is directed to a compound of formula (1) as defined above, wherein n₁ is 2.

According to another particular embodiment, the present invention is directed to a compound of formula (I) as defined above, wherein R1 is a iodine atom, a radionuclide, or Sn(R)₃, where R is a (C₁-C₆) alkyl group, and more particularly R1 is a iodine atom.

According to another particular embodiment, the present invention is directed to a compound of formula (I) as defined above, wherein
R3 represents:
- a -(CH₂CH₂O)ₙ₂-(CH₂)₂-X group, where n₂ is an integer varying from 3 to 7,
- a -CH₂-Y group with Y representing a -C=C-H, a -N₃ or a -Ar-(CH₂)ₙ₆-X group with
   Ar being 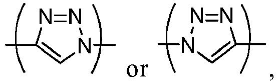 n₆ representing an integer varying from 1 to 4, and
X is a halogen atom, a radionuclide or a -OSO₂R' group, where R' is a CF₃, CH₃, *t*-Bu, Ph, *p*-NO₂Ph, *p*-BrPh or *p*-CH₃Ph group.

According to another particular embodiment, the present invention is directed to a compound of formula (Ia) 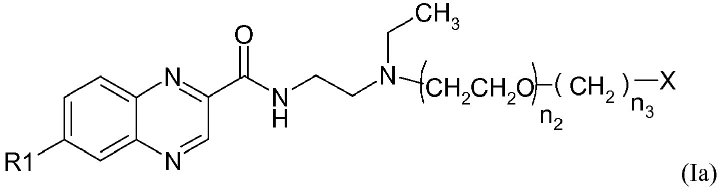 in which
R1 represents Sn(R)₃, B(OH)₂, B(OR)₂, halogen, NO₂, a radionuclide or a -N⁺(R)₃ group, where R is a (C₁-C₆) alkyl group,
n₂ is an integer varying from 1 to 12,
n₃ is an integer varying from 1 to 4, and
X is a halogen atom, a radionuclide or a -OSO₂R' group, where R' is a CF₃, CH₃, *t*-Bu, Ph, *p*-NO₂Ph, *p*-BrPh or *p*-CH₃Ph group,
and their addition salts with pharmaceutically acceptable acids.

According to said particular embodiment, the present invention is more particularly directed to the compounds of formula (Ia), wherein n₂ varies between 3 and 7 and n₃ is 2.

According to said same particular embodiment, the present invention is more particularly directed to the compounds of formula (Ia), wherein R1 represents a iodine atom, an optionally labelled iodine atom or Sn(R)₃, where R is a (C₁-C₆) alkyl group, and more particularly R1 represents a iodine atom, and X represents an optionally labelled fluorine atom or a -OSO₂R' group, where R' is a CF₃, a *t*-Bu or a CH₃ group, and more particularly a CH₃ group.

According to another particular embodiment, the present invention is directed to a compound of formula (Ib) 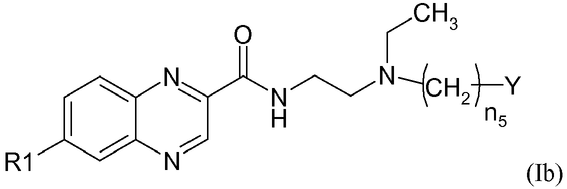 in which
R1 represents Sn(R)₃, B(OH)₂, B(OR)₂, a halogen atom, NO₂, a radionuclide or a -N⁺(R)₃ group, where R is a (C₁-C₆)alkyl group,
n₅ represents an integer varying from 1 to 4, and
Y represents a -C=C-H, a -N₃ or a -Ar-(CH₂)ₙ₆-(OCH₂CH₂)ₙ₇-X group with
Ar being 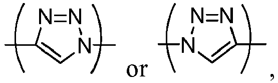 n₆ representing an integer varying from 1 to 4, n₇ an integer varying from 0 to 12 and X a halogen, a radionuclide or a -OSO₂R' group, where R' is a CF₃, CH₃, *t*-Bu, Ph, *p*-NO₂Ph, *p*-BrPh or *p*-CH₃Ph,
and their addition salts with pharmaceutically acceptable acids.

According to said particular embodiment, the present invention is more particularly directed to the compounds of formula (Ib) wherein n₅ is 1 and Y represents a -C=C-H, a -N₃ or a -Ar-(CH₂)ₙ₆-X group with
Ar being 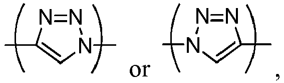 n₆ representing an integer varying from 1 to 4 and X a halogen, a radionuclide or a OSO₂R' group, where R' is a CF₃, CH₃, *t*-Bu, Ph, *p*-NO₂Ph, *p*-BrPh or *p*-CH₃Ph.

According to said particular embodiment, the present invention is more particularly directed to the compounds of formula (Ib) wherein R1 represents a iodine atom, a radionuclide or Sn(R)₃, where R is a (C₁-C₆) alkyl group, and more particularly R1 represents a iodine atom, and Y represents a -C=C-H or a -Ar-(CH₂)ₙ₆-X group with
Ar being 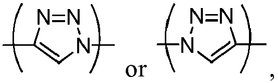 n₆ representing an integer varying from 1 to 4 and X representing an optionally labelled fluorine atom or a -OSO₂R' group, where R' is a CF₃ or a CH₃ group, and more particularly a CH₃ group.

Moreover formulae (I), (Ia) and (Ib) encompass the radiotracers themselves, which are the agents for imaging and/or for targeted radionuclide therapy of melanoma which typically encompass only one radioisotope, but also the corresponding precursors, more particularly the compounds which are not yet labelled, *i.e.* their non-radio labelled precursors.

According to another aspect, the invention is directed to labelled compounds of formula (I), (Ia) ore (Ib).

The following table presents various possible combinations of meaning for R1 and X:

| **R1** | **X** |
|---|---|
| I | F |
| I | ¹⁸F |
| Sn(R)₃ | F |
| any isotpe of I, such as ¹³¹I and ¹²⁵I | F |
| I | -OSO₂R' |
| halogen other than I | F |
| ⁷⁶Br^{, 77}Br^{, 210}At^{, 211}At | F |

According to another aspect, a subject-matter of the invention is a product chosen from the compounds of formula (I), (Ia) or (Ib) and in particular a labelled compound of formula (I), (Ia) or (Ib) as defined above for the diagnosis and/or the treatment of melanoma.

In the context of the present invention, the term:
- "halogen" is understood to mean chlorine, fluorine, bromine, iodine or astatine,
- "(C₁-C₆)alkyl" as used herein refers to C₁-C₆ normal, secondary, or tertiary saturated hydrocarbon. Examples are, but are not limited to, methyl, ethyl, 1-propyl, 2-propyl, 1-butyl, 2-methyl-1-propyl (*i*-Bu), 2-butyl (*s*-Bu), 2-methyl-2-propyl (*t*-Bu). In a particular embodiment, the term also includes (C₁-C₆)haloalkyl group, which is a (C₁-C₆)alkyl group bearing at least one halogen,
- "Ph" means a phenyl group.

Within the meaning of the present invention, the term "radionuclide" is understood to mean an isotope of natural or artificial origin which demonstrates radioactive properties. The radionuclide can be a radioisotope chosen from iodine-123, iodine-124, iodine-125, iodine-131, bromine-75, bromine-76, bromine-77, fluorine-18, astatine-210 or astatine-211.

According to a particular embodiment, R1 and X can independently and preferably not simultaneously represent a radionuclide selected from iodine-123, iodine-124, iodine-125, iodine-131, astatine-211 and fluorine-18.

Moreover, the term "labelled" as used herein means "radio labelled" and is more precisely directed to a compound comprising at least one radionuclide, *i. e.* a radioactive nuclide.

According to a specific embodiment, the compound of formula (I), (Ia) or (Ib) comprises at least one iodine atom and more particularly said iodine atom being comprised in the R1 group.

The following particular embodiments form part of the present invention, which may be considered independently or which may be combined together, their addition salts with pharmaceutically acceptable acids being encompassed:
- the compound of formula (I), (Ia) or (Ib) wherein R1 represents an optionally labelled iodine atom or Sn(R)₃, where R is a (C₁-C₆) alkyl group, and more particularly R1 represents an optionally labelled iodine atom,
- the compound of formula (I), (Ia) or (Ib), wherein n₁ is 2, and
- the compound of formula (I), (Ia) or (Ib), wherein R2 is an ethyl group.

According to a preferred embodiment of the present invention, the compound is chosen from:
- N-(12-ethyl-1-fluoro-3,6,9-trioxa-12-azatetradecan-14-yl)-6-iodoquinoxaline-2-carboxamide **(1),**
- *N*-(12-ethyl-1 -methanesulfonyloxy-3,6,9-trioxa-12-azatetradecan-14-yl)-6-iodoquinoxaline-2-carboxamide **(2),**
- *N-*(12-ethyl-1-fluoro-3,6,9-trioxa-12-azatetradecan-14-yl)-6-(tributylstannyl)quinoxaline-2-carboxamide **(4),**
- *N-*(24-ethyl-1-fluoro-3,6,9,12,15,18,21-heptaoxa-24-azahexacosan-26-yl)-6-iodoquinoxaline-2-carboxamide **(5),**
- *N-*(24-ethyl-1-methanesulfonyloxy-3,6,9,12,15,18,21-heptaoxa-24-azahexacosan-26-yl)-6-iodoquinoxaline-2-carboxamide **(6),**
- *N-*[2-[(*N-*ethyl)-[[1-(2-fluoroethyl)-1*H-*(1,2,3)triazol-4-yl]methyl]amino]ethyl]-6-iodoquinoxaline-2-carboxamide **(7),**
- *N-*[2-[(*N*-ethyl)-(*N*-prop-2-ynyl)amino]ethyl]-6-iodoquinoxaline-2-carboxamide **(8),**
- and their pharmaceutically acceptable salts, more particularly their hydrochlorides, or bases.

The compounds of formulae (I), (Ia) and (Ib) can comprise one or more asymmetric carbon atoms. They can thus exist in the form of enantiomers or of diastereoisomers. These enantiomers, diastereoisomers and their mixtures, including the racemic mixtures, form part of the invention.

The pharmaceutically acceptable salts of the compounds of formulae (I), (Ia), and (Ib) include the addition salts with pharmaceutically acceptable acids, such as inorganic acids, for example hydrochloric, hydrobromic, phosphoric or sulphuric acid and organic acids, such as acetic, trifluoroacetic, propionic, oxalic, succinic, fumaric, malic, tartaric, citric, ascorbic, maleic, glutamic, benzoic, toluenesulphonic, methanesulphonic, stearic and lactic acid.

The compounds of formulae (I), (Ia) and (Ib) or their salts can form solvates (namely hydrates); the invention includes such solvates.

A further aspect of the invention pertains to a radiolabelled compound of formula (I), (Ia) or (Ib) as described above, wherein said radiolabelling occurs by means of a radionuclide which can be a radioisotope chosen among iodine-123, iodine-124, iodine-125, iodine-131, bromine-75, bromine-76, bromine-77, fluorine-18, astatine-210 or astatine-211, and particularly is chosen among fluorine-18, iodine-123, iodine-124, iodine-125 et iodine-131.

When the compounds in accordance with the present invention of formulae (I), (Ia) and (Ib) are used for medical imaging purposes, one of R1 and X is preferably a radionuclide possessing γ or β⁺ emission which can be detected according to conventional radioimaging techniques, for example scintigraphic imaging by single photon emission computed tomography (SPECT) and by positron emission tomography (PET).

Such a radionuclide possessing γ or β⁺ emission advantageously exhibits an optimum energy for the measurement by means of a γ camera or PET camera. Mention may in particular be made, as radionuclides acceptable for medical imaging, of iodine-123, iodine-124, iodine-125, iodine-131, bromine-75, bromine-76, bromine-77 and fluorine-18.

Iodine-123 is particularly suitable for SPECT scintigraphic diagnosis and fluorine-18 or iodine-124 for a PET scintigraphic diagnosis.

When the compounds of the invention of formulae (I), (Ia) and (Ib) are used for therapeutic purposes, R1 is preferably a radionuclide possessing α, β⁻ or Auger electron emission. The radionuclides suitable in this context, capable of providing a cytotoxic effect, can be chosen from iodine-131, iodine-125, astatine-210 and astatine-211.

Iodine-131 is particularly suitable for an application in the treatment of melanoma in targeted radionuclide therapy. Furthermore, iodine-125, due to its Auger electron emission, can be used in targeted radiotherapy provided that it is internalized in the cell.

Compounds according to the general formulae (I), (Ia) and (Ib) can be prepared by condensation of a compound according to the general formula (II) 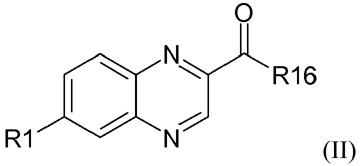 wherein:
R1 is as defined above,
R16 is a -OH group, a halogen, a -OR17 group or a -OCOR18 group,
   wherein
   R17 is a (C₁-C₆)alkyl, an aryl or heteroaryl group, for example chosen from benzene, pentafluorobenzene, *p*-nitrobenzene, triazole, benzotriazole, 7-azabenzotriazole and succinimide and more particularly is a (C₁-C₄)alkyl or a *p*-nitrophenyl group,
   R18 is a (C₁-C₆)alkyl, an aryl or a (C₁-C₄)alkoxy group as ethoxy for example,
with a diamine, according to the general formula (III)

H₂N-(CH₂)ₙ₁-NR2R3 (III)

wherein:
n1, R2 are as defined above and R3 is as defined above and is further extended to the groups where X is a OR", R" meaning an alcohol protecting group such as tert-butyl dimethylsilyl (TBDMS).

Condensation of compound (II) with diamine (III) can be performed according to general methods described by Montalbetti, C. A. G. N.; Falque, V. Amide bond formation and peptide coupling. Tetrahedron 2005, 61, 10827-10852.

Preferentially, the amide bond formation between an ester (II) and diamine (III) can be achieved in the presence or not of trimethylaluminium under reflux of anhydrous solvents such as dichloromethane, toluene or every other appropriate solvent. This condensation can also be achieved at room temperature in tetrahydrofuran, in the case of activated *p*-nitrophenyl ester.

Preferentially, the amide bond formation between an acyl halide (II), particularly an acyl chloride (II), and diamine (III) can be achieved in any inert dry solvents, in presence or not of a non-nucleophilic tertiary amine as base to trap the formed HCl. These reactions can also be accelerated in presence of pyridine, *N*,*N-*dimethylaminopyridine or metallic zinc used as catalyst.

These syntheses processes form part of the present invention.

Diamines, according to the general formula (III) as defined above can be prepared preferentially *via* intermediates containing a protective group on the primary amine.

The term "protective group" for the functional amino group discussed above are described in Greene and Wuts, "Protective groups in Organic Synthesis", John Wiley & Sons (1991**),** the entire teachings of which are incorporated into this application by reference. The person skilled in the art can select, using no more than routine experimentation, suitable protecting groups for use in the disclosed synthesis, including protecting groups other than those described below, as well as conditions for applying and removing the protecting groups.

According to a particular embodiment, the protective group can be a phthalimide group or a *tert*-butoxycarbonyl (Boc) group. In this case, diamines, according to the general formula (III), can be obtained after removing of the protective group and liberation of the primary amine, at room temperature or under reflux, by acidic hydrolysis of corresponding intermediates, containing a phthalimide or a Boc group or for phthalimide derivatives by reaction of those compounds with aqueous or ethanolic hydrazine solution.

The compounds of formulae (I), (Ia) or (Ib) in which R2 or R3 is a hydrogen can preferably be prepared by condensation of the amine (III) with an ester (II) in which R₁₇ is a *p*-nitrophenyl group. This reaction can preferably be carried out in tetrahydrofuran at ambient temperature.

The compounds of formula (II), wherein R₁₆ is a -OH group or a -OR₁₇ group, can be synthesized according to the methods as described in WO2009/95872.

The starting compounds of formula (III) are commercially available or are described in the literature, or can be synthetized in accordance with methods which are described therein or which are known to the man skilled in the art.

Radioiodination of compounds, according to the general formula (I), (Ia) and (Ib) by iodine-123, iodine-124, iodine-125 or iodine-131, can be performed using several differents methods (Dewandjee, M. K. Radioiodination: theory, practice, and biomedical application. Kluwer academic publishers, Norwell, USA, 1992**).** For example, it can be achieved by an exchange, in acidic conditions, between the non radioactive iodinated molecule and an alkaline radioactive halide. The exchange can be carried out, for example under a temperature in a range of 100 to 200 °C, using an aqueous solution of compounds, according to the general formula (I), (Ia) or (Ib) and radioactive halide as [¹²⁵I]NaI in buffered medium or in acetic acid, in the presence or not of catalysts such as, for example, copper(II) sulfate. Radiolabelling can also be performed between a trialkylstannane precursor of compounds, according to the general formula (I), (Ia) or (Ib) and an alkaline halide as [¹²⁵I]NaI or [¹³¹I]NaI in the presence of an oxidative agent as chloramine-T, peracetic acid, hydrogen peroxide or in the presence or not of an acid as hydrochloric acid, acetic acid or an acid buffer solution, preferentially at room temperature and in an appropriate solvent.

Radiofluorination strategies and nature of the source of fluorine-18 for nucleophilic [¹⁸F] fluorination.

Fluorine-18-labelling of compound according to the general formula (I), (Ia), or (Ib) can be performed by nucleophilic substitutions with [¹⁸F]fluoride, in order to maximize the specific radioactivities. Nucleophilic substitutions (aliphatic and aromatic) with [¹⁸F]fluoride will usually be performed either on an immediate precursor of the target molecule (direct labelling using a one-step process) or on an indirect precursor followed by one or more chemical steps leading to the target radiotracer.

There is no particular restriction on the nature of the sources of [¹⁸F]fluorides to be used in this reaction, and any sources of [¹⁸F]fluorides conventionally used in reactions of this type may equally be used here, provided that it has no adverse effect on other parts of the molecule. Examples of suitable sources of [¹⁸F]fluorides include: alkali metal [¹⁸F]fluorides, such as sodium [¹⁸F]fluoride, potassium [¹⁸F]fluoride, cesium [¹⁸F]fluoride; ammonium [¹⁸F]fluoride, tetraalkylammonium [¹⁸F]fluorides, such as tetrabutylammonium [¹⁸F]fluoride. Of these, the alkali metal [¹⁸F]fluorides, and notably a potassium [¹⁸F]fluoride, are preferred. The source of [¹⁸F]fluorides may be activated by the presence of a ligand able to complex the counter cationic species of the source of [¹⁸F]fluorides. The ligand may be notably a cyclic or polycyclic multidentate ligand. Examples of suitable ligands include notably crown ethers such as 1,4,7,10,13-pentaoxacyclooctadecane (18C6) or cryptands such as 4,7,13,16,21,24-hexaoxa-1,10-diazabicyclo-[8,8,8]hexacosane sold under the name K222^{®}. Preferably, the source of [¹⁸F]fluoride is an alkaline metal [¹⁸F]fluoride-cryptate complex, notably a potassium [¹⁸F]fluoride-cryptate complex, preferably the potassium [¹⁸F]fluoride - 4,7,13,16,21,24-hexaoxa-1,10-diazabicyclo-[8,8,8]hexacosane ([¹⁸F]KF/K222^{®}). The complex [¹⁸F]KF/K222^{®} may be prepared by any conventional methods, for example as disclosed in Dollé, F.; Dolci, L.; Valette, H.; Hinnen, F.; Vaufrey, F.; Guenther, I.; Fuseau, C.; Coulon, C.; Bottlaender, M.; Crouzel, C. Synthesis and Nicotinic Acetylcholine Receptor in vivo Binding Properties of 2-Fluoro-3-[2(S)-2-azetidinylmethoxy]pyridine: A New Positron Emission Tomography Ligand for Nicotinic Receptors. J. Med. Chem. 1999, 42, 2251-2259.

### Typical example 1: One-step nucleophilic [¹⁸F]fluorination

The simplest pathway proposed for the labelling with fluorine-18 of [¹⁸F]fluoroalkyl derivatives (compounds of formula (I), (Ia)) is a one-step radiochemical process, involving a direct substitution of an appropriated atom or group (a leaving part) by [¹⁸F]fluorine.

The best leaving-groups are the weakest bases, which is consistent with the principle that stable species make better leaving-groups. Iodide is usually the best leaving-group of the halides and fluoride the poorest. The sulphonic ester groups, such as the triflate (CF₃SO₃-), tosylate *(*p-MeC₆H₄SO₃-), mesylate (CH₃SO₃-), brosylate (*p*-BrC₆H₄SO₃-), nosylate (*m*-NO₂C₆H₄SO₃-), or *tert*-butylsulfonate (*t*-BuSO3-) are better leaving-groups than halides, leading to the following ranking order: RSO₃ > I > Br > Cl > F. Of course, fluorine, in spite of its excellent leaving-group ability, is seldom used in fluorine-18 chemistry because of obvious isotopic dilution, leading to low specific radioactivity.

Aliphatic nucleophilic substitutions are usually performed under basic or neutral conditions, with a large assortment of solvents possible. Indeed, the effects of the solvent on SN₂-type reactions depend on the charge dispersal in the transition state compared to the one in the reactants. However, in radiofluorinations with [¹⁸F]fluoride the solubility of the reactants appears to play a larger role in solvent choice than the solvent effects on reaction rates. The most common solvents are the polar aprotic ones, such as acetonitrile, in which the [¹⁸F]fluoride salt ([¹⁸F]KF, [¹⁸F]CsF, [¹⁸F]Bu₄NF or [¹⁸F]KF/K222^{®} complex for example) and the organic precursors for labelling show good solubility. The reaction is often performed at reflux temperature of the solvent for a few minutes.

### Typical example 2:

Two-step process involving a nucleophilic [¹⁸F]fluorination followed by a (multi)deprotection step.

A typical pathway also often proposed for the labelling with fluorine-18 of [¹⁸F]fluoroalkyl derivatives (compounds of formula (I), (Ia) or (Ib)) is a two-step radiochemical process, involving first a direct substitution of an appropriated atom or group (a leaving part) by [¹⁸F]fluoride (as described above) followed by a (multi)deprotection step. (Multi)deprotection step is usually performed under basic or acidic conditions and depends on the chemical nature of the protective groups. Heating the reaction mixture for a few minutes may be required.

### Typical example 3:

Two-step process involving a nucleophilic [¹⁸F]fluorination followed by a coupling step.

One robust and reliable pathway frequently proposed for the labelling with fluorine-18 of compounds of formula (I),) or (Ib) is a two-step radiochemical process, involving first the preparation of an appropriate azido, alcyne, halogeno or better sulphonyloxy [¹⁸F]reagent. The radiosynthesis of azido, alcyne, n-bromo-, n-tosyloxy- and n-mesyloxy- 1-[¹⁸F]fluoroalkanes (n = 1-3) from the corresponding bifunctional alkanes by nucleophilic aliphatic substitution with no-carrier-added [¹⁸F]fluoride as for example its activated [¹⁸F]KF/K222® complex. Then, coupling with alkyne ((I), (Ib)) is the second step.
Réf : Ackermann et al, J. Label Compd. Radiopharm, 2011, 54, 260-266, Glaser et al, Bioconjugate Chem, 2007, 18, 989-993, Smith et al, J. Med. Chem,, 2008, 51, 8057-8067.

### Typical example 4:

One-step nucleophilic [¹⁸F]fluorination in the aromatic series, i.e. in the case were the precursor is linked to an aromatic ring, for example R1 is NO₂ or a halogen atom.

The simplest pathway proposed for the labelling with fluorine-18 of [¹⁸F]fluoroaryl derivatives is a one-step radiochemical process, involving a direct substitution of an appropriated atom or group (a leaving part) by [¹⁸F]fluoride.

Leaving-groups include the halogens and particularly the nitro group (with the following accepted order of decreasing leaving-group ability: F > NO₂ > Cl > Br and I). However, this depends greatly on the nature of the nucleophile and with [¹⁸F]fluoride, the trimethylammonium group is often a better alternative, when available. Indeed, aryltrimethylammonium salts, which are relatively stable and easy to handle, tend to be more reactive than compounds with a neutral leaving-group and usually require milder conditions and give higher radiochemical yields in [¹⁸F]fluoride substitution. They are also particularly convenient because of their superior separation from the reaction product, the neutral aryl [¹⁸F]fluoride, using HPLC or an SPE-cartridge, due to the large differences in physical-chemical properties. The possible side-reaction consisting of fluorodemethylation on the trimethylammonium group, leading to volatile [¹⁸F]fluoromethane, is a limiting factor in the use of this leaving-group. Again, fluorine, in spite of its excellent leaving-group ability, is seldom used in fluorine-18 chemistry because of obvious isotopic dilution, leading to low specific radioactivity.

Aromatic nucleophilic radiofluorinations are usually performed in aprotic polar solvents, such as DMSO, sulfolane or dimethylacetamide, and often under basic conditions (due for example to the presence of Kryptofix-222^{®}/potassium carbonate). Completion of the [¹⁸F]fluoride incorporation often requires moderate to high temperatures (100 °C-170 °C) for ten to thirty minutes. Microwave technology can be a successful application here resulting in improved yields and shorter reaction times.

The invention also relates to a method for preparing a [¹⁸F]radiolabelled compound according to the general formula (I), (Ia) or (Ib) comprising at least the steps of i) reacting a dedicated precursor for labelling with a source of [¹⁸F]fluoride in an appropriate solvent and optionally ii) recovering the obtained [¹⁸F]radiolabelled compound according to the general formula (I), (Ia) or (Ib).

The method for preparing the [¹⁸F]radiolabelled compounds of general formula (I), (Ia) or (Ib) can take place over a wide range of temperatures, and the precise reactions temperatures are not critical to the invention. In general, it is convenient to carry out the reactions at a temperature of from about 50 °C to about 150°C. The time required for the reactions may also vary widely, depending on many factors, notably the reactions temperatures and the nature of the reagents. However, provided that the reactions are effected under the preferred conditions outlined above, a period of from about 5 minutes to about 15 minutes will usually suffice. The [¹⁸F]radiolabelled compound of general formula (I), (Ia) or (Ib) thus prepared may be recovered from the reaction mixture by conventional means. For example, the compounds may be recovered by filtration on a prepacked separation column. Additionally, the product can, if desired, be further purified by various well known techniques, such as chromatography techniques, notably High Performance Liquid Chromatography (HPLC).

The trialkylstannane precursor compounds of the compounds of formulae (I), (Ia) and (Ib) also form part of the present invention. They are referred to as compounds of formula (V): 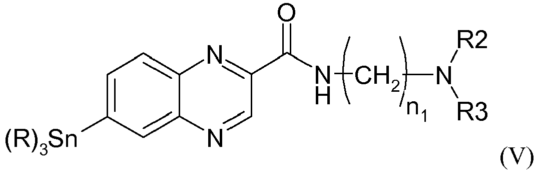 in which n1, R, R2 and R3 have the same meaning as above.

The following examples illustrate the invention.

All reagents and solvents were purchased in the following commercial suppliers: Sigma Aldrich, Acros Organics, Carlo Erba and SDS. All solvents were dried using common techniques. Unless otherwise noted, moisture sensitive reactions were conducted under dry argon. Thin layer chromatography (TLC) was performed on silica gel 60 F254 plates or neutral aluminium oxide 60 F254 plates (Merck or SDS) and visualized with UV light and/or developed with iodine, nynhydrin or potassium permanganate. Flash chromatography (fc) was performed on silica gel 60A normal phase, 35-70 µm (Merck or SDS) or neutral aluminium oxide 90 standardized, 63-200 µm (Merck, column chromatographic adsorption analysis acc. to Brockmann). Uncorrected melting points (mp) were recorded on an electrothermal IA9300 (capillary) or a Reichert-Jung-Koffler apparatus. NMR spectra (400 or 200 MHz for 1H and 100 or 50 MHz for 13C) were recorded on a Bruker Avance 400 or 200 instrument; 19F NMR spectra (470 MHz) were recorded on a Bruker DRX 500 apparatus using tetrafluorotoluene as internal reference (-63 ppm). δ were expressed in ppm. Infrared spectra (IR) were recorded on a FTIR Nicolet Impact 410, a FT Vector 22 or a Nicolet IS10 with attenuated total reflectance (ATR) accessory. Electron impact mode mass spectra were obtained on a 5989A instrument (Agilent Technologies) or HP5890 series II chromatograph coupled to HP5985B mass spectrometer. The analysis of samples was performed in CH3CN at a final concentration of 1 pmol/µL. Electrospray ionization mass spectra (ESI-MS) were recorded on TSQ 7000 ThermoQuest Finnigam (Les Ulis, France) or Esquire-LC (Bruker Daltonics, Wissenbourg, France) spectrometers. The analysis of samples was performed in CH3CN at a final concentration of 1 pmol/µL, CH3OH/H2O (1/1, v/v, containing 1% HCOOH) or CH3CN/H2O (1/1, v/v, containing 1% HCOOH) in positive mode and CH3OH/H2O (1/1, v/v, containing 1% NH4OH) in negative mode, at a final concentration of 8-12 pmol/µL. Each ESI-MS spectrum was recorded by averaging of 10 spectra. Microanalyses were performed by Analytical Laboratory of the CNRS (Vernaison, France) for the elements indicated.

### EXAMPLE 1: Synthesis of N-(12-ethyl-1-methanesulfonyloxy-3,6,9-trioxa-12-azatetradecan-14-yl)-6-iodoquinoxaline-2-carboxamide (2) (Precursor of [¹⁸F]1) and N-(12-ethyl-1-fluoro-3,6,9-trioxa-12-azatetradecan-14-yl)-6-iodoquinoxaline-2-carboxamide (1).

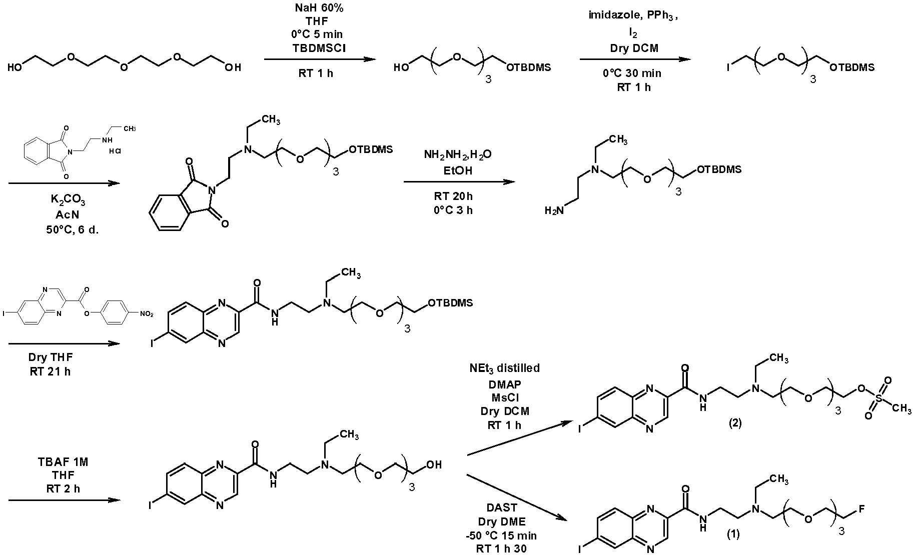

**Step 1: Synthesis of 2,2,3,3-tetramethyl-4,7,10,13-tetraoxa-3-silapentadecan-15-ol.** A solution of sodium hydride (60% in mineral oil, 2.94 g, 73.5 mmol) in anhydrous tetrahydrofuran (135 mL) was stirred at room temperature for 5 min under argon. Dried tetraethylene glycol (8.80 mL, 51.0 mmol) was added dropwise at 0°C and the reaction mixture was stirred for 5 min before addition of *tert*-butyldimethylsilyl chloride (12.00 g, 79.6 mmol). After return back to room temperature, the resulting mixture was stirred for 1 h and the reaction was quenched by addition of water (150 mL). The mixture was extracted with ethyl acetate (3x100 mL). The organic layers were combined, washed successively with a saturated aqueous ammonium chloride solution (150 mL) and brine (150 mL), dried on magnesium sulfate, filtered and evaporated under vacuum. The crude product was purified by chromatography (SiO₂ pad, EtOAc/pentane, 75/25, v/v then 50/50, v/v and finally CH₂Cl₂/EtOH, 98/2, v/v) to yield the expected compound (6.42 g, 20.8 mmol) as a yellow oil. Yield 41%. *R*_{f} (SiO₂, EtOAc/pentane, 75/25, v/v) 0.16. IR (ATR diamond accessory) v 778, 837, 1109, 1255, 1472, 2859, 2930, 3300-3600 cm⁻¹.¹H NMR (200 MHz, CDCl₃) δ (ppm) 0.05 (s, 6H), 0.88 (s, 9H), 2.63 (brs, 1H), 3.65 (m, 16H). ¹³C NMR (50 MHz, CDCl₃) δ (ppm) -5.2 (2C), 26.0 (3C), 61.8, 62.8, 70.4-70.7 (4C), 72.6, 72.7. MS m/z 309 (1, [M+H]⁺), 163 (26), 147 (22), 133 (23), 119 (46), 103 (55), 89 (100), 87 (9), 75 (93), 73 (61), 59 (29).
**Step 2: Synthesis of 15-iodo-2,2,3,3-tetramethyl-4,7,10,13-tetraoxa-3-silapentadecane.** To a stirred solution of alcohol obtained in the preceding step (5.00 g, 16.2 mmol) in anhydrous dichloromethane (150 mL) were successively added imidazole (1.44 g, 21.1 mmol), triphenylphosphine (5.53 g, 21.1 mmol) and iodine (5.35 g, 21.1 mmol) at 0°C under argon. The mixture was stirred at 0°C for 30 min, then at room temperature for 1 h. A 10% aqueous sodium bisulfite solution (170 mL) was added and the mixture was forcefully stirred for 2 min. After decantation, the organic layer was collected and the aqueous layer was extracted with dichloromethane (3×80 mL). The organic layers were combined, dried on magnesium sulfate, filtered and evaporated under vacuum. The obtained solid was suspended in n-pentane (150 mL) and the mixture was stirred at room temperature for 45 min before filtration. The precipitate was washed with n-pentane (30 mL). The filtrate was evaporated under vacuum and the oily residue was purified by chromatography (SiO₂ pad, CH₂Cl₂/EtOH, 98/2, v/v) to give the expected compound as a pale yellow oil (5.62 g, 13.4 mmol). Yield 83%. *R*_{f} (SiO₂, CH₂Cl₂/EtOH, 98/2, v/v) 0.25. IR (ATR diamond accessory) v 778, 837, 1109, 1256, 1472, 2858, 2928 cm⁻¹.¹H NMR (200 MHz, CDCl₃) δ (ppm) 0.04 (s, 6H), 0.87 (s, 9H), 3.24 (t, 2H, *J* = 7.0 Hz), 3.53 (t, 2H, *J =* 5.4 Hz), 3.64 (m, 8H), 3.73 (m, 4H). ¹³C NMR (50 MHz, CDCl₃) δ (ppm) -5.2 (2C), 3.0, 18.4, 26.0 (3C), 62.8, 70.3-70.8 (4C), 72.1, 72.7. MS m/z 361 (3), 155 (100).
**Step 3: Synthesis of *N-*(16-ethyl-2,2,3,3-tetramethyl-4,7,10,13-tetraoxa-16-aza-3-silaoctadecan-18-yl)phthalimide.** To a stirred solution of *N*-[2-(ethylamino)ethyl]-1*H-*phthalimide hydrochloride (3.04 g, 12.0 mmol) (Jones, J. Med. Chem., 1973, 16, 537-542) in anhydrous acetonitrile (80 mL) were successively added potassium carbonate (1.65 g, 12.0 mmol) and compound obtained at the preceding step (5.00 g, 12.0 mmol) under argon. The mixture was heated at 50°C for 6 days. Back to room temperature, a saturated aqueous sodium carbonate solution (200 mL) was added and the resulting solution was extracted with dichloromethane (3×150 mL). The organic layers were combined, dried on magnesium sulfate, filtered and evaporated under vacuum. The oily residue was purified by chromatography (SiO₂, EtOAc) to afford the expected compound (4.38 g, 8.61 mmol) as a pale yellow oil. Yield 72%. *R*_{f} (SiO₂, EtOAc) 0.25. IR (NaCl) v 837, 1105, 1254, 1396, 1468, 1592, 1713, 2858, 2900 cm⁻¹. ¹H NMR (200 MHz, CDCl₃) δ (ppm) 0.04 (s, 6H), 0.87 (s, 9H), 0.97 (t, 3H, *J* = 7.1 Hz), 2.61 (q, 2H, *J=* 7.1 Hz), 2.74 (m, 4H), 3.57 (m, 12H), 3.76 (m, 4H), 7.70 (m, 2H), 7.80 (m, 2H). ¹³C NMR (50 MHz, CDCl₃) δ (ppm) -5.2 (2C), 12.0, 18.4, 26.0 (3C), 36.2, 48.4, 51.5, 53,0, 62.8, 69.8, 70.5, 70.6, 70.7, 70.8, 72.7, 123.2 (2C), 132.3 (2C), 133.9 (2C), 168.4 (2C). ESI-MS m/z 509.4 [M+H]⁺.
**Step 4: Synthesis of *N-*16-ethyl-2,2,3,3-tetramethyl-4,7,10,13-tetraoxa-16-aza-3-silaoctadecan-18-amine.** To a stirred solution of compound of the preceding step (1.50 g, 2.95 mmol) in ethanol (150 mL) was added hydrazine monohydrate (1.43 mL, 29.5 mmol). The mixture was stirred at room temperature for 20 h and cooled at 0°C for 3 h. The white precipitate was filtered and washed with ice-cold ethanol (50 mL) and dichloromethane (50 mL) and the filtrate was evaporated under vacuum. The residue was suspended in dichloromethane (20 mL) and the remaining precipitate was filtered again and washed with dichloromethane (20 mL). The last steps were repeated until no more white solid appeared after evaporation of the filtrate. The expected compound (1.11 g, 2.95 mmol) was obtained as a pale yellow oil and use without further purification. Yield quant. *R*_{f} (Al₂O₃, CH₂Cl₂/EtOH, 8/2, v/v) 0.32. IR (ATR diamond accessory) v 834, 1107, 1252, 1472, 1574, 2857, 2929, 3100-3300 cm⁻¹.¹H NMR (200 MHz, CDCl₃) δ (ppm) 0.05 (s, 6H), 0.88 (s, 9H), 1.01 (t, 3H, *J=* 7.1 Hz), 2.68 (m, 8H), 3.55 (m, 12H), 3.72 (m, 2H). ¹³C NMR (50 MHz, CDCl₃) δ (ppm) -5.2 (2C), 11.9, 26.0 (3C), 39.7, 48.8, 52.7, 56.4, 62.8, 70.5 (5C), 72.7.
**Step 5: Synthesis of *N-*(16-ethyl-2,2,3,3-tetramethyl-4,7,10,13-tetraoxa-16-aza-3-silaoctadecan-18-yl)-6-iodoquinoxaline-2-carboxamide.** To a stirred suspension of the activated ester 4-nitrophenyl 6-iodoquinoxaline-2-carboxylate (305 mg, 0.72 mmol) (Denoyer, J. Chromatogr. B. Analyt. Technol. Biomed. Life Sci. 2008, 875, 411-418.) in anhydrous tetrahydrofuran (7 mL) was added a solution of compound obtained in the preceding step (430 mg, 1.14 mmol) in anhydrous tetrahydrofuran (8 mL) under argon. The mixture was stirred at room temperature for 21 h and the solvent was then evaporated under vacuum. The obtained residue was dissolved in dichloromethane (15 mL) and a 1 N aqueous sodium hydroxide solution was added (45 mL). After decantation, the aqueous layer was extracted with dichloromethane (3X15 mL). The organic layers were combined, washed with a 5% aqueous sodium carbonate solution (2×45 mL), dried on magnesium sulfate, filtered and evaporated under vacuum. The oily residue was then chromatographed (SiO₂, CH₂Cl₂/EtOH, 9/1, v/v) to provide the expected compound (459 mg, 0.70 mmol) as a pale yellow oil. Yield 96%. *R*_{f} (SiO₂, CH₂Cl₂/EtOH, 9/1, v/v) 0.30. IR (NaCl) v 834, 1108, 1253, 1353, 1387, 1472, 1523, 1592, 1676, 1714, 2857, 2928, 3025, 3300-3400 cm⁻¹. ¹H NMR (200 MHz, CDCl₃) δ (ppm) 0.04 (s, 6H), 0.87 (s, 9H), 1.08 (t, 3H, *J* = 7.1 Hz), 2.78 (m, 6H), 3.60 (m, 16H), 7.82 (d, 1H, *J=* 8.8 Hz), 8.07 (dd, 1H, *J* = 1.8, 8.8 Hz), 8.46 (m, 1H), 8.60 (d, 1H, *J=* 1.8 Hz), 9.63 (s, 1H). ¹³C NMR (50 MHz, CDCl₃) δ (ppm) -5.2 (2C), 12.1, 18.4, 26.0 (3C), 37.4, 48.7, 52.9, 53.0, 62.7, 70.7 (5C), 72.7, 98.0, 130.9, 138.6, 139.7 (2C), 144.2, 144.4, 144.7, 163.0. ESI-MS m/z 661.3 [M+H]⁺.
**Step 6: Synthesis of *N-*(12-ethyl-1-hydroxy-3,6,9-trioxa-12-azatetradecan-14-yl)-6-iodoquinoxaline-2-carboxamide.** To a stirred solution of compound obtained in the preceding step (173 mg, 0.26 mmol) in tetrahydrofuran (8 mL) was added a 1 M solution oftetrabutylammonium fluoride in tetrahydrofuran (393 µL, 0.39 mmol). The mixture was then stirred at room temperature for 2 h. The reaction was quenched by addition of a saturated aqueous sodium hydrogencarbonate solution (80 mL) followed by distilled water (40 mL) and then ethyl acetate (40 mL). After decantation, the aqueous layer was extracted with ethyl acetate (3×40 mL). The organic layers were combined, washed with brine (40 mL), dried on magnesium sulfate, filtered and evaporated under vacuum. The residue obtained was chromatographed (SiO₂, CH₂Cl₂/EtOH, 95/5 to 80/20, v/v) to give the expected compound (132 mg, 0.24 mmol) as a pale yellow oil. Yield 93%. *R*_{f} (SiO₂, CH₂Cl₂/EtOH, 80/20, v/v) 0.26. IR (NaCl) v 1125, 1353, 1473, 1527, 1592, 1671, 2871, 3300-3400 cm⁻¹. ¹H NMR (200 MHz, CDCl₃) δ (ppm) 1.10 (t, 3H, *J=* 7.1 Hz), 2.82 (m, 6H), 3.60 (m, 16H), 7.83 (d, 1H, *J* = 8.8 Hz), 8.06 (dd, 1H, *J* = 1.8, 8.8 Hz), 8.57 (m, 1H), 8.59 (d, 1H, *J =* 1.8 Hz), 9.62 (s, 1H). ¹³C NMR (50 MHz, CDCl₃) δ (ppm) 11.7, 37.3, 48.9, 52.9, 53.1, 61.7, 69.7, 70.3-70.6 (4C), 72.7, 98.0, 130.9, 138.6, 139.7 (2C), 144.2, 144.4, 144.7, 163.2. ESI-MS m/z 547.2 [M+H]⁺.
**Step 7: Synthesis of *N-*(12-ethyl-1-fluoro-3,6,9-trioxa-12-azatetradecan-14-yl)-6-iodoquinoxaline-2-carboxamide (1).** To a solution of compound obtained in the preceding step (100 mg, 183 µmol) in anhydrous dimethoxyethane (800 µL) was added (diethylamino)sulfur trifluoride (49 µL, 366 µmol) at -50°C under argon atmosphere. The mixture was stirred at -50°C for 15 min, then at room temperature for 90 min, and poured into a saturated aqueous sodium hydrogencarbonate solution (10 mL). The mixture was extracted with ethyl acetate (3×10 mL). The organic layers were combined, dried on magnesium sulfate, filtered and evaporated under vacuum. The residue was purified by chromatography (SiO₂, EtOAc/EtOH/NH₄OH, 85/15/0.5, v/v/v) to yield compound (1) as a yellow oil (68 mg, 120 µmol). Yield 68%. *R*_{f} (SiO₂, EtOAc/EtOH, 85/15, v/v) 0.30. IR (ATR diamond accessory) v 1046, 1107, 1352, 1473, 1523, 1592, 1669, 2868, 2916, 3300-3400 cm⁻¹. ¹H NMR (200 MHz, CDCl₃) δ (ppm) 1.07 (t, 3H, *J* = 7.1 Hz), 2.65 (q, 2H, *J=* 7.1 Hz), 2.77 (m, 4H), 3.61 (m, 12H), 3.69 (m, 2H), 4.53 (td, 2H, ²*J*_{H-F} = 47.7 Hz, *J=* 4.1 Hz), 7.81 (d, 1H, *J* = 8.8 Hz), 8.07 (dd, 1H,*J*= 1.8, 8.8 Hz), 8.42 (m, 1H), 8.60 (d, 1H, *J* = 1.8 Hz), 9.63 (s, 1H). ¹³C NMR (50 MHz, CDCl₃) δ (ppm) 11.5, 37.1, 48.9, 52.8 (2C), 70.8 (6C), 83.2 (¹*J*_{C-F} = 169 Hz), 98.1, 131.0, 138.6, 139.5, 139.7, 144.1, 144.4, 144.7, 163.3.¹⁹F NMR (CDCl₃) δ (ppm) -223.1. ESI-MS m/z 549.2 [M+H]⁺.
**Step 8: Synthesis of *N-*(12-ethyl-1-methanesulfonyloxy-3,6,9-trioxa-12-azatetradecan-14-yl)-6-iodoquinoxaline-2-carboxamide (2).** To a solution of compound obtained in step 6 (80 mg, 0.146 mmol), distilled triethylamine (25 µL, 0.176 mmol, 1.2 eq.), and 4-(dimethylamino)pyridine (1.8 mg, 14.6 µmol) in anhydrous dichloromethane (2.5 mL) was added methanesulfonyl chloride (14 µL, 0.176 mmol) under argon. The mixture was stirred at room temperature for 1 h before addition of an aqueous saturated solution of sodium hydrogencarbonate (25 mL). After decantation, the aqueous layer was extracted with dichloromethane (3x25 mL). The organic layers were combined, dried on magnesium sulfate, filtered and evaporated under vacuum. The obtained residue was purified by chromatography (SiO₂, CH₂Cl₂ → CH₂Cl₂/EtOH 9/1, v/v) to give compound **(2)** (85 mg, 0.136 mmol) as a yellow oil. Yield 93%. *R_{f} (*SiO₂*,* CH₂Cl₂/EtOH 9/1, v/v) 0.37. IR (ATR diamond accessory) v 751, 917, 1106, 1171, 1349, 1473, 1525, 1592, 1668, 2849, 2916, 3300-3400 cm⁻¹. ¹H NMR (200 MHz, CDCl₃) δ (ppm) 1.12 (t, 3H, *J* = 7.1 Hz), 2.85 (m, 6H), 3.06 (s, 3H), 3.60 (m, 12H), 3.73 (m, 2H), 4.36 (m, 2H), 7.83 (d, 1H, *J =* 8.8 Hz), 8.06 (dd, 1H, *J =* 1.8, 8.8 Hz), 8.53 (m, 1H), 8.60 (d, 1H, *J =* 1.8 Hz), 9.62 (s, 1H). ¹³C NMR (50 MHz, CDCl₃) δ (ppm) 11.7, 37.2, 37.8, 48.9, 52.9, 53.0, 69.1, 69.3, 70.5 (5C), 98.1, 130.9, 138.6, 139.6, 139.8, 144.1, 144.4, 144.7, 163.2. ESI-MS m/z 625.3 [M+H]⁺.
**Step 9: Synthesis of *N*-(12-ethyl-1-fluoro-3,6,9-trioxa-12-azatetradecan-14-yl)-6-iodoquinoxaline-2-carboxamide dihydrochloride salt (3), dihydrochloride salt of (1).** This experiment was carried out in a glove compartment, under an argon atmosphere. To a solution of compound **(1)** (10 mg, 18.2 µmol) in anhydrous dichloromethane (1 mL) was added a 2.0 N hydrochloric acid solution in anhydrous diethylether (2 mL) under argon. After 5 min, the solvent was evaporated under a flux of argon. The residue was suspended in anhydrous diethylether (2 mL) and dried under a flux of argon to give compound **(3),** the dihydrochloride salt of **(1),** as a very hygroscopic yellow solid. IR (ATR diamond accessory) v 409, 1048, 1130, 1355, 1475, 1538, 1592, 1674, 2953, 3300-3400 cm⁻¹; ¹H NMR (200 MHz, CDCl₃) δ 1.07 (t, 3H, *J* = 7.1 Hz), 3.37 (m, 6H), 3.65 (m, 10H), 3.77 (m, 2H), 4.02 (q, 2H, *J =* 6.2 Hz), 4.54 (td, 2H, ²*J*_{H-F} = 47.7 Hz, *J =* 4.2 Hz), 7.96 (d, 1H, *J =* 8.9 Hz), 8.08 (dd, 1H, *J =* 1.9, 8.9 Hz), 8.59 (d, 1H, *J =* 1.6 Hz), 9.28 (m, 1H), 9.56 (s, 1H); ¹⁹F NMR (CDCl₃) δ-222.76; ESI-MS m/z 549.1 [M+H]⁺; Anal. (C₂,H₃₀FIN₄O₄, 2HCl, 5H₂O) calculated: C: 35.46; H: 5.95; N: 7.88; found: C: 35.81; H: 6.23; N: 8.04.

### EXAMPLE 2: Synthesis of N-(12-ethyl-1-fluoro-3,6,9-trioxa-12-azatetradecan-14-yl)-6-(tributylstannyl)quinoxaline-2-carboxamide (4) (Precursor for radiosynthesis of [¹³¹I]1).

To a solution of compound (1), obtained in step 7 of example 1 (50 mg, 91.2 µmol) and hexabutylditin (102 µL, 0.20 mmol), in anhydrous toluene (2.5 mL) was added bis(triphenylphosphine)palladium (II) dichloride (3.2 mg, 4.6 µmol) under argon. The mixture was stirred at reflux for 5.5 h. After cooling to room temperature, the mixture was filtered on Celite® 545 and the pad was washed with ethyl acetate (3×10 mL). The filtrate was evaporated under vacuum. The obtained residue was purified by chromatography (SiO₂, CH₂Cl₂/EtOH 1/0, v/v → CH₂Cl₂/EtOH 9/1, v/v) to give compound the expected compound (20 mg, 28.1 µmol) as a yellow oil. Yield 31%. *R_{f}* (SiO₂, CH₂Cl₂/EtOH 9/1, v/v) 0.36. IR (NaCl) v 1044, 1105, 1260, 1351, 1463, 1524, 1674, 2877, 2925, 3300-3400 cm⁻¹. ¹H NMR (200 MHz, CDCl₃) δ (ppm) 0.88 (t, 9H, *J* = 7.1 Hz), 1.25 (m, 21H), 2.90 (m, 6H), 3.64 (m, 14H), 4.54 (td, 2H, *J =* 4.1 Hz, ²*J*_{H-F} = 47.7), 7.91 (d, 1H, *J=* 8.2 Hz), 8.05 (d, 1H, *J=* 8.2 Hz), 8.30 (m, 1H, ³*J*_{H-117Sn/119Sn =} 41.7 Hz), 8.56 (s, 1H), 9.63 (s, 1H). ¹³C NMR (50 MHz, CDCl₃) δ 10.0, 11.4, 13.7, 27.4 (²*J*_{C-117Sn/119Sn} = 56 Hz), 29.1 (*³J*_{*C-11*7Sn/119Sn} = 21 Hz), 37.0, 49.1, 52.9, 53.1, 70.6-70.9 (6C), 83.2 (¹*J*_{C-F} = 169 Hz), 128.2, 137.8, 138.1, 140.5, 143.0, 143.4, 143.7, 149.1, 163.8. ESI-MS m/z 713.40 [M+H]⁺.

### EXAMPLE 3: Synthesis of N-(24-ethyl-1-methanesulfonyloxy-3,6,9,12,15,18,21-heptaoxa-24-azahexacosan-26-yl)-6-iodoquinoxaline-2-carboxamide (6) (Precursor of [¹⁸F]5) and N-(24-ethyl-1-fluoro-3,6,9,12,15,18,21-heptaoxa-24-azahexacosan-26-yl)-6-iodoquinoxaline-2-carboxamide (5).

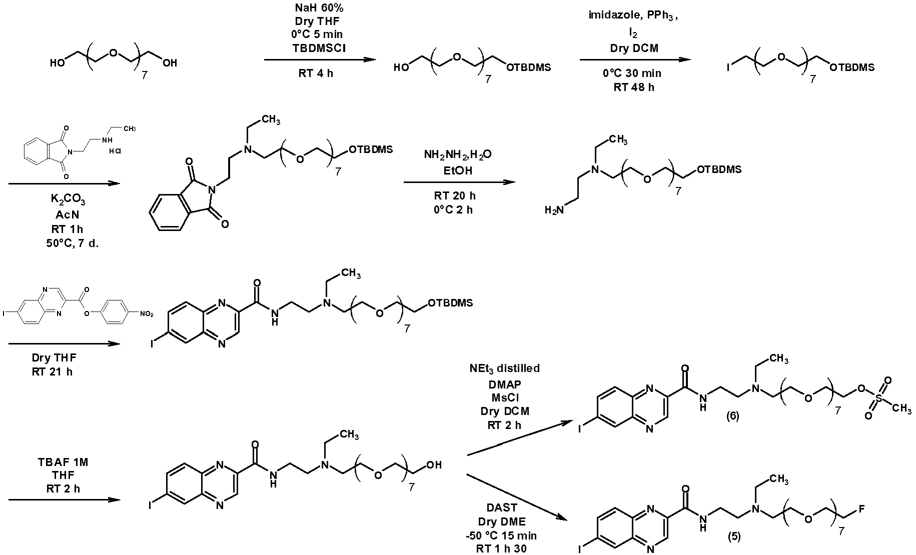

**Step 1: Synthesis of 2,2,3,3-tetramethyl-4,7,10,13,16,19,22,25-octaoxa-3-silaheptacosan-27-ol.** This compound was synthesized according to the procedure described in step 1 of example 1, starting from octaethylene glycol (6.01 g, 16.2 mmol), sodium hydride (60% in mineral oil, 0.91 g, 22.7 mmol) and *tert*-butyldimethylsilyl chloride (3.67 g, 24.3 mmol). Reaction time: 4 h at room temperature; the purification was performed using column chromatography (SiO₂, EtOAc/EtOH, 95/5, v/v then 8/2, v/v) to give the expected compound (5.32 g, 11.0 mmol) as a yellow oil. Yield 68%. *R_{f}* (SiO₂, EtOAc/EtOH, 95/5, v/v) 0.21. IR (NaCl) v 778, 836, 1106, 1254, 2873, 2927, 3100-3600 cm⁻¹.¹H NMR (200 MHz, CDCl₃) δ (ppm) 0.00 (s, 6H), 0.83 (s, 9H), 3.58 (m, 32H). ¹³C NMR (50 MHz, CDCl₃) δ (ppm) -5.2 (2C), 18.4, 26.0 (3C), 61.7, 62.7, 70.4-70.6 (12C), 72.6, 72.7. MS m/z 281 (13), 251 (13), 207 (53), 189 (12), 177 (9), 163 (34), 159 (27), 147 (38), 145 (15), 133 (45), 119 (49), 117 (13), 115 (27), 103 (49), 101 (21), 89 (100), 87 (22), 75 (46), 73 (80), 71 (16), 59 (35).
**Step 2: Synthesis of 27-iodo-2,2,3,3-tetramethyl-4,7,10,13,16,19,22,25-octaoxa-3-silaheptacosane.** This compound was synthesized according to the procedure described for in step 2 of example 1, starting from the compound of the preceding step (2.00 g, 4.13 mmol), imidazole (365 mg, 5.36 mmol), triphenylphosphine (1.41 g, 5.36 mmol) and iodine (1.36 g, 5.36 mmol). Reaction time: 2 days at room temperature; the purification was performed using column chromatography (SiO₂, EtOAc) to give the expected compound as a pale yellow oil (1.89 g, 3.18 mmol). Yield 77%. *R_{f}*(SiO2_{,} EtOAc) 0.30. IR (NaCl) v 778, 835, 1108, 1253, 2859, 2927 cm⁻¹. ¹H NMR (200 MHz, CDCl₃) δ (ppm) 0.06 (s, 6H), 0.89 (s, 9H), 3.26 (t, 2H, *J=* 7.0 Hz), 3.55 (t, 2H, *J* = 5.5 Hz), 3.65 (m, 24H), 3.70 (m, 4H). ¹³C NMR (50 MHz, CDCl₃) δ (ppm) -5.2 (2C), 3.0, 18.4, 26.0 (3C), 62.8, 70.3-70.7 (12C), 72.1, 72.7. MS m/z 361 (2), 331 (1), 317 (9), 287 (6), 243 (8), 207 (12), 199 (28), 189 (8), 159 (16), 155 (100), 133 (5), 117 (13), 115 (15), 103 (21), 101 (11), 89 (16), 87 (13), 75 (14), 73 (60), 71 (13), 59 (18).
**Step 3: Synthesis of *N*-(28-ethyl-2,2,3,3-tetramethyl-4,7,10,13,16,19,22,25-octaoxa-28-aza-3-silaheptacosan-30-yl)phthalimide.** To a stirred solution of *N-*[2-(ethylamino)ethyl]-1*H-*phthalimide hydrochloride (489 mg, 1.92 mmol) (Jones, J. Med. Chem., 1973, 16, 537-542) in anhydrous acetonitrile (15 mL) was added potassium carbonate (530 mg, 3.84 mmol) under argon. The reaction mixture was stirred at room temperature for 1 h before addition of the compound of the preceding step (1.14 g, 1.92 mmol). The mixture was heated at 50°C for 7 days, cooled to room temperature and partitioned between a saturated aqueous sodium carbonate solution (50 mL) and dichloromethane (30 mL). After decantation, the aqueous layer was extracted with dichloromethane (3×30 mL). The organic layers were combined, dried on magnesium sulfate, filtered and evaporated under vacuum. The oily residue was purified by chromatography (SiO₂, CH₂Cl₂/EtOH, 95/5, v/v) to afford the expected compound (854 mg, 1.25 mmol) as a pale yellow oil. Yield 65%. *R_{f}* (SiO₂, CH₂Cl₂/EtOH, 95/05, v/v) 0.32. IR (ATR diamond accessory) v 838, 1112, 1255, 1397, 1436, 1469, 1717, 2862, 2929 cm⁻¹. ¹H NMR (200 MHz, CDCl₃) δ (ppm) 0.05 (s, 6H), 0.88 (s, 9H), 0.99 (t, 3H, *J =* 7.1 Hz), 2.66 (q, 2H, *J =* 7.5 Hz), 2.73 (m, 4H), 3,56 (m, 4H), 3.63 (m, 24H), 3.76 (m, 4H), 7.70 (m, 2H), 7.83 (m, 2H). ¹³C NMR (50 MHz, CDCl₃) δ (ppm) -5.2 (2C), 12.0, 18.4, 26.0 (3C), 36.1, 48.5, 51.4, 53.0, 62.8, 70.6 (13C), 72.7, 123.2 (2C), 132.3 (2C), 133.9 (2C), 168.4 (2C); ESI-MS m/z 685.5 [M+H]⁺.
**Step 4: Synthesis of 28-ethyl-2,2,3,3-tetramethyl-4,7,10,13,16,19,22,25-octaoxa-28-aza-3-silaheptacosan-30-amine.** This compound was synthesized according to the procedure described for in step 4 of example 1, starting from the compound of the preceding step (607 mg, 0.89 mmol) and hydrazine monohydrate (430 µL, 8.86 mmol). Reaction time: 20 h at room temperature to give the expected compound (492 mg, 0.89 mmol) as a pale yellow oil which was used without further purification. Yield quant. *R_{f}* (Al₂O₃, CH₂Cl₂/EtOH, 9/1, v/v) 0.23. IR (NaCl) v 837, 1108, 1252, 1299, 1472, 1648, 2859, 2929, 3100-3600 cm⁻¹. ¹H NMR (200 MHz, CDCl₃) δ (ppm) 0.04 (s, 6H), 0.87 (s, 9H), 1.00 (t, 3H, *J =* 7.1 Hz), 2.61 (m, 8H), 3.51 (t, 2H, *J =* 6.3 Hz), 3.53 (t, 2H, *J =* 5.4Hz), 3.63 (m, 24H), 3.74 (t, 2H, *J* = 5.4 Hz). ¹³C NMR (50 MHz, CDCl₃) δ (ppm) -5.2 (2C), 12.0, 18.4, 26.0 (3C), 39.9, 48.7, 53.0, 56.9, 62.8, 70.1-70.6 (13C), 72.7.
**Step 5: Synthesis of *N*-(28-ethyl-2,2,3,3-tetramethyl-4,7,10,13,16,19,22,25-octaoxa-28-aza-3-silaheptacosan-30-yl)-6-iodoquinoxaline-2-carboxamide.** This compound was synthesized according to the procedure described in step 5 of example 1, starting from the compound of the preceding step (450 mg, 0.81 mmol) and the activated ester 4-nitrophenyl 6-iodoquinoxaline-2-carboxylate (220 mg, 0.52 mmol). Reaction time: 21 h at room temperature; the purification was performed using column chromatography (SiO₂, CH₂Cl₂/EtOH, 95/5, v/v then 9/1, v/v) to provide the expected coompound (435 mg, 0.52 mmol) as a pale yellow oil. Yield 99%. *R_{f}* (SiO₂, CH₂Cl₂/EtOH, 9/1, v/v) 0.28. IR (NaCl) v 835, 1107, 1252, 1352, 1473, 1527, 1592, 1677, 2860, 2927, 3300-3400 cm⁻¹. ¹H NMR (200 MHz, CDCl₃) δ (ppm) 0.03 (s, 6H), 0.85 (s, 9H), 1.04 (t, 3H, *J=* 7.1 Hz), 2.65 (q, 2H, *J=* 7.1 Hz), 2.74 (m, 4H), 3.61 (m, 32H), 7.80 (d, 1H, *J=* 8.8 Hz), 8.05 (dd, 1H, *J* = 1.8, 8.8 Hz), 8.41 (m, 1H), 8.57 (d, 1H, *J* = 1.8 Hz), 9.61 (s, 1H). ¹³C NMR (50 MHz, CDCl₃) δ (ppm) -5.2 (2C), 12.2, 18.4, 26.0 (3C), 37.6, 48.7, 52.9, 53.0, 62.8, 70.2-70.6 (13C), 72.7, 97.9, 130.9, 138.6, 139.7 (2C), 144.2, 144.4, 144.7, 163.0. ESI-MS m/z 837.4 [M+H]⁺.
**Step 6: Synthesis of *N*-(24-ethyl-1-hydroxy-3,6,9,12,15,18,21-heptaoxa-24-azahexacosan-26-yl)-6-iodoquinoxaline-2-carboxamide.** This compound was synthesized according to the procedure described in step 6 of example 1, starting from the compound of the preceding step (425 mg, 0.51 mmol) and tetrabutylammonium fluoride 1 M in tetrahydrofuran (762 µL, 0.76 mmol). Reaction time: 1.5 h at room temperature; the purification was performed using column chromatography (SiO₂, CH₂Cl₂/EtOH, 9/1 then 8/2, v/v) to afford the expected compound as a yellow oil (273 mg, 0.38 mmol). Yield 75%. *R_{f} (*SiO₂*,* CH₂Cl₂/EtOH, 8/2, v/v) 0.36. IR (ATR diamond accessory) v 1127, 1353, 1474, 1529, 1593, 1675, 2800-3000, 3100-3600 cm⁻¹. ¹H NMR (200 MHz, CDCl₃) δ (ppm) 1.06 (t, 3H, *J* = 7.1 Hz), 2.66 (q, 2H, *J=* 7.1 Hz), 2.76 (m, 4H), 2.91 (m, 1H), 3.60 (m, 32H), 7.81 (d, 1H, *J*= 8.8 Hz), 8.05 (dd, 1H, *J* = 1.8, 8.8 Hz), 8.44 (m, 1H), 8.58 (d, 1H, *J=* 1.8 Hz), 9.61 (s, 1H). ¹³C NMR (50 MHz, CDCl₃) δ (ppm) 12.1, 37.5, 48.7, 52.9, 53.0, 61.7, 70.0-70.6 (13C), 72.7, 98.0, 130.9, 138.6, 139.7 (2C), 144.2, 144.4, 144.7, 163.0. ESI-MS m/z 723.3 [M+H]⁺.
**Step 7: Synthesis of *N-*(24-ethyl-1-fluoro-3,6,9,12,15,18,21-heptaoxa-24-azahexacosan-26-yl)-6-iodoquinoxaline-2-carboxamide (5).** This compound was synthesized according to the procedure described in step 7 of example 1, starting from the compound of the preceding step (94 mg, 0.13 mmol) and (diethylamino)sulfur trifluoride (34.4 µL, 0.26 mmol). Reaction time: 15 min at -50°C and 1.5 h at room temperature; the purification was performed using column chromatography (SiO₂, EtOAc/EtOH/NH₄OH, 8/2/0.5, v/v/v) to yield the expected compound as a yellow oil (63 mg, 86.9 µmol). Yield 67%. *R_{f}* (SiO₂, EtOAc/EtOH, 5/5, v/v containing 0.5% of NH₄OH) 0.32. IR (ATR diamond accessory) v 1129, 1353, 1474, 1527, 1593, 1675, 2800-3000, 3300-3400 cm⁻¹. ¹H NMR (200 MHz, CDCl₃) δ (ppm) 1.07 (t, 3H, *J =* 7.1 Hz), 2.67 (q, 2H, *J =* 7.1 Hz), 2.79 (m, 4H), 3.61 (m, 30H), 4.55 (td, 2H, ²*J*_{H-F} = 47.8 Hz, *J =* 4.1 Hz), 7.83 (d, 1H, *J =* 8.8 Hz), 8.08 (dd, 1H, *J =* 1.6, 8.8 Hz), 8.46 (m, 1H), 8.61 (d, 1H, *J =* 1.6 Hz), 9.64 (s, 1H). ¹³C NMR (50 MHz, CDCl₃) δ (ppm) 11.5, 37.0, 48.8, 52.9, 53.0, 70.6 (14C), 83.3 (¹*J_{C-F} =* 163 Hz), 98.1, 131.0, 138.6, 139.7, 139.7, 144.1, 144.4, 144.7, 163.3. ¹⁹F NMR (470 MHz, CDCl₃) δ (ppm) -223.2. ESI-MS m/z 725.2 [M+H]⁺. Anal. (C₂₉H₄₆O₈IN₄F, 3H₂O) calculated: C: 44.73; H: 6.73; N: 7.20; found: C: 44.84; H: 7.16, N: 5.82).
**Step 8: Synthesis of *N*-(24-ethyl-1-methanesulfonyloxy-3,6,9,12,15,18,21-heptaoxa-24-azahexacosan-26-yl)-6-iodoquinoxaline-2-carboxamide (6).** This compound was synthesized according to the procedure described in step 8 of example 1, starting from the compound obtained in step 6 of the present example (91 mg, 0.126 mmol), distilled triethylamine (21 µL, 0.151 mmol), 4-(dimethylamino)pyridine (1.5 mg, 13.0 µmol) and methanesulfonyl chloride (12 µL, 0.151 mmol). Reaction time: 2 h at room temperature; the purification was performed using column chromatography (SiO₂, CH₂Cl₂ CH₂Cl₂/EtOH 9/1, v/v) to give the expected compound (95 mg, 0.120 mmol) as a yellow oil. Yield 95%. *R_{f}* (SiO₂, CH₂Cl₂/EtOH, 8/2, v/v) 0.50. IR (ATR diamond accessory) v 733, 923, 1128, 1176, 1354, 1474, 1593, 1527, 1675, 2800-3000, 3300-3400 cm⁻¹. ¹H NMR (200 MHz, CDCl₃) δ (ppm) 1.04 (t, 3H, *J =* 7.1 Hz), 2.64 (q, 2H, *J =* 7.1 Hz), 2.73 (m, 4H), 3.06 (s, 3H), 3.61 (m, 28H), 3.73 (t, 2H, *J =* 4.5 Hz), 4.35 (t, 2H, *J =* 4.5 Hz), 7.80 (d, 1H, *J* = 8.8 Hz), 8.05 (dd, 1H, *J* = 1.3, 8.8 Hz), 8.41 (m, 1H), 8.57 (d, 1H, *J* = 1.3 Hz), 9.61 (s, 1H). ¹³C NMR (50 MHz, CDCl₃) δ (ppm) 12.2, 37.6, 37.8, 48.7, 52.9, 53.0, 69.1, 69.4, 70.2-70.6 (13C), 98.0, 130.9, 138.6, 139.6, 139.7, 144.2, 144.4, 144.7, 163.0. ESI-MS m/z 801.3 [M+H]⁺.

### EXAMPLE 4: Synthesis of N-[2-[(N-ethyl)-(N-prop-2-ynyl)amino]ethyl]-6-iodoquinoxaline-2-carboxamide (8) (Precursor of [¹⁸F]7) and N-[2-[(N-ethyl)-[[1-(2-fluoroethyl)-1H-(1,2,3)triazol-4-yl]methyl] amino] ethyl] -6-iodoquinoxaline-2-carboxamide (7).

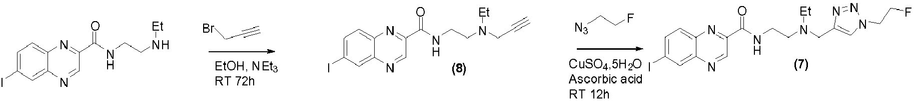

**Step 1: Synthesis of *N*-[2-[(*N*-ethyl)-(*N*-prop-2-ynyl)amino]ethyl]-6-iodoquinoxaline-2-carboxamide (8).** To a solution of compound *N-*(2-(ethylamino)ethyl)-6-iodoquinoxaline-2-carboxamide (Denoyer, J. Chromatogr. B. Analyt. Technol. Biomed. Life Sci. 2008, 875, 411-418.) (500 mg, 1.35 mmol) in anhydrous ethanol (10 mL) were successively added propargyl bromide (122 µL, 1.62 mmol) and triethylamine (187 µL, 1.35 mmol). The mixture was stirred at room temperature for 72 h before addition of water (33 mL). The resulting solution was then extracted with dichloromethane (3×33 mL). The organic layers were combined, dried on magnesium sulfate, filtered and evaporated under *vacuum*. The obtained residue was purified by chromatography (Al₂O₃, CH₂Cl₂/EtOH, 99/1, v/v) to give the expected compound (522 mg, 1.28 mmol) as a beige solid. Yield 95%. *R_{f}* (Al₂O₃, CH₂Cl₂/EtOH, 99/1, v/v) 0.48. mp 135-137°C. IR (KBr) v 1047, 1176, 1355, 1474, 1534, 1670, 2967, 3000-3600 cm⁻¹. ¹H NMR (200 MHz, CDCl₃) δ (ppm) 1.12 (t, 3H, *J =* 7.1 Hz), 2.23 (t, 1H, *J =* 2.3 Hz), 2.67 (q, 2H, *J =* 7.1 Hz), 2.84 (t, 2H, *J =* 6.0 Hz), 3.52 (d, 2H, *J =* 2.3 Hz), 3.62 (q, 2H, *J=* 6.0 Hz), 7.78 (d, 1H, *J =* 8.8 Hz), 8.02 (dd, 1H, *J =* 1.9, 8.8 Hz), 8.32 (m, 1H), 8.54 (d, 1H, *J =* 1.9 Hz), 9.59 (s, 1H). ¹³C NMR (50 MHz, acetone-d₆) δ (ppm) 13.2, 37.8, 41.9, 48.1, 52.8, 74.5, 79.3, 98.3, 131.9, 139.2, 140.3, 140.6, 145.2, 145.4, 145.6, 163.5. ESI-MS m/z 409.1 [M+H]⁺.
**Step 2: Synthesis of** *N-*[2-[(*N-*ethyl)-[[1-(2-fluoroethyl)-1*H-*(1,2,3)triazol-4-**yl]methyl]amino]ethyl]-6-iodoquinoxaline-2-carboxamide (7).** Cesium fluoride (336 mg, 2.21 mmol) was dried by azeotropic distillation with anhydrous acetonitrile (4×7 mL) at 90°C, under *vacuum*. Back to room temperature, a solution of p-toluenesulfonate-2-azidoethyl (176 mg, 0.73 mmol), synthesized according to the procedure described by Demko (Org. Lett., 2001, 3, 4091-4094.), in a mixture of anhydrous *tert*-amyl alcohol and acetonitrile (7.4 mL, 1/1, v/v) was added under argon. The mixture was stirred at 100°C for 2 h to yield 2-fluoro-1-azidoethane. Back to room temperature, a 1.0 M aqueous pentahydrate copper (II) sulfate solution (370 µL), a 1.0 M aqueous ascorbic acid solution (370 µL) and compound **(8)** of the preceding step (300 mg, 0.73 mmol) were successively added. The mixture was stirred at room temperature for 12 hours before addition of water (5 mL) and a 2.0 M aqueous sodium carbonate solution (5 mL). The solution was extracted with dichloromethane (4×10 mL). The organic layers were combined, dried on magnesium sulfate, filtered and evaporated under *vacuum*. The residue was chromatographed (Al₂O₃, CH₂Cl₂/EtOH, 99/1, v/v) to give the expected compound (311 mg, 0.63 mmol) as a brown oil. Yield 86%. *R_{f}*(Al₂O₃, CH₂Cl₂/EtOH, 99/1, v/v) 0.41. IR (CCl₄) v 1145, 1354, 1388, 1437, 1473, 1522, 1659, 1673, 2837, 2971, 3246, 3386 cm⁻¹. ¹H NMR (200 MHz, CDCl₃) δ (ppm) 1.12 (t, 3H, *J =* 7.1 Hz), 2.64 (q, 2H, *J =* 7.1 Hz), 2.76 (t, 2H, *J* = 6.1 Hz), 3.59 (q, 2H, *J* = 6.1 Hz), 3.88 (s, 2H), 4.57 (td, 2H, ³*J*_{H-F}= 27.5 Hz, *J =* 4.6 Hz), 4.71 (td, 2H, ²*J*_{H-F}= 51.0 Hz, *J* = 4.6 Hz), 7.65 (s, 1H), 7.83 (d, 1H, *J =* 8.8 Hz), 8.05 (dd, 1H, *J =* 1.8, 8.8 Hz), 8.40 (m, 1H), 8.57 (d, 1H, *J =* 1.8 Hz), 9.60 (s, 1H). ¹³C NMR (50 MHz, CDCl₃) δ (ppm) 12.1, 37.2, 47.6, 48.1, 50.4 (d, ²*J*_{C-F} = 20 Hz), 51.5, 81.5 (d, *¹J*_{C-F} = 172 Hz), 98.0, 123.5 (d, ³*J*_{C-F} = 14 Hz), 130.7, 138.4, 139.4, 139.6, 144.0, 144.2, 144.5, 145.3, 162.9.¹⁹F NMR (470 MHz, CDCl₃) δ (ppm) -223.21. ESI-MS m/z 498.0 [M+H]⁺.
**Step 3: Synthesis of *N*-[2-[(*N*-ethyl)-[[1-(2-fluoroethyl)-1*H*-(1,2,3)triazol-4-yl] methyl] amino] ethyl]-6-iodoquinoxaline-2-carboxamide trihydrochloride salt (9), trihydrochloride salt of (7).** To a stirred solution of compound (7) (200 mg, 0.40 mmol) in anhydrous dichloromethane (5 mL) was added under argon a 2.0 N hydrochloric acid solution in anhydrous diethylether (10 mL). After 10 min, the solvent was evaporated under *vacuum*. The residue was suspended in anhydrous diethylether (10 mL) and the mixture was stirred at room temperature for 14 h. The precipitate was then filtered and dried under reduced pressure to give the trihydrochloride salt of (7) (209 mg, 0.34 mmol) as a very hygroscopic yellow solid. Yield 86%. mp 98-100°C. IR (KBr) v 1040, 1169, 1356, 1474, 1534, 1670, 2600-3600 cm⁻¹.¹H NMR (200 MHz, DMSO-d₆) δ (ppm) 1.34 (t, 3H, *J =* 7.0 Hz), 3.15 (m, 2H), 3.30 (m, 2H), 3.84 (m, 2H), 4.55 (m, 2H), 4.70 (s, 2H), 4.89 (td, 2H, ³*J*_{H-F}= 20.9 Hz, *J=* 4.4 Hz), 5.36 (m, 2H), 7.92 (d, 1H, *J =* 8.8 Hz), 8.24 (dd, 1H, *J =* 1.6, 8.8 Hz), 8.47 (s, 1H), 8.62 (d, 1H, *J* = 1.6 Hz), 9.44 (m, 2H), 11.08 (brs, 1H). ¹³C NMR (50 MHz, DMSO-d₆) δ (ppm) 8.6, 34.1, 45.4, 47.3, 50.2 (d, ²*J*_{C-F} = 19 Hz), 50.3, 81.8 (d, *¹J*_{C-F} = 167 Hz), 99.6, 127.6 (d, ³*J*_{C-F} = 12 Hz), 130.7, 136.1, 137.5, 138.9, 139.8, 143.6, 144.3, 144.4, 163.6. ¹⁹F NMR (470 MHz, DMSO-d₆) δ (ppm) -223.95. ESI-MS m/z 498.1 [M+H]⁺. Anal. (C₁₈H₂₁OIN₇F, 3HCl, H₂O) calculated: C: 34.61; H: 4.20; N: 15.70; found: C: 37.74; H: 4.61; N: 15.49.

The chemical structures and physical data of some unlabelled compounds of formulae (Ib) of the invention are illustrated in the following Table I.

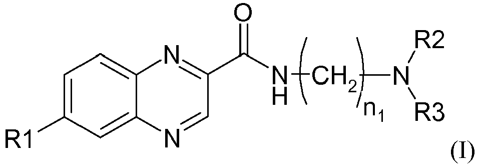

**Table I**

| **N°** | 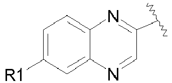 | **n₁** | **R2** | **R3** | **Salt** | **aspect** |
|---|---|---|---|---|---|---|
| **1** | 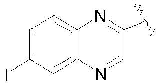 | 2 | Et | 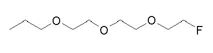 | - | oil |
| **3** | | | | | 2 HCl | Yellow solid |
| **2** | 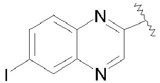 | 2 | Et | 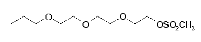 | - | Yellow oil |
| **4** | 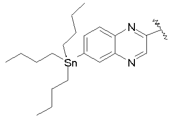 | 2 | Et | 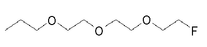 | - | Yellow oil |
| **5** | 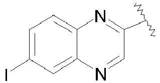 | 2 | Et | 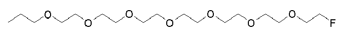 | - | Yellow oil |
| **6** | 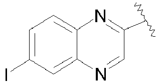 | 2 | Et | 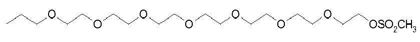 | - | Yellow oil |
| **7** | 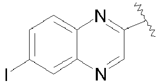 | 2 | Et | 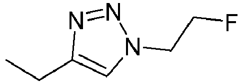 | | Brown oil |
| **9** | | | | | 3HCl | Yellow solid |
| **8** | 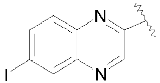 | 2 | Et | 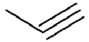 | - | Beige solid |

### - Et denotes an ethyl radical

Some radio labelling examples are reported below.

### Materials for Radiolabelling with iodine-125 or iodine-131

[¹²⁵I]NaI (3.7 GBq/mL, 643.8 MBq/mg) was purchased from PerkinElmer Life and Analytical Sciences (331 Treble Cove Road, Billerica, MA 01862, US) as a no-carrier-added solution in reductant free 1.0 × 10⁻⁵ M aqueous sodium hydroxide solution (pH 8-11). [¹³¹I]NaI (66.21 GBq/mL, 712.82 GBq/mg) was purchased from PerkinElmer Life and Analytical Sciences (331 Treble Cove Road, Billerica, MA 01862, US) as a 0.1 M sodium hydroxide solution (pH 12-14). Extrelut® and citrate buffer solution (pH = 4) were purchased from Merck (Darmstadt, Germany). The radio TLC plates (Merck neutral aluminium oxide 60F254 or Fluka, aluminium oxide on TLC-PET foils, 60Å, 0.2 mm) were developed with CH₂Cl₂/EtOH (98/2, v/v) and measured on an AMBIS 400 (Scanalytics, CSPI, San Diego, CA, USA). Semi-preparative RP-HPLC purification of **[¹²⁵I](7)** was performed on a system including a Shimadzu LC 6A pump, SLC 6B controller, a CR5A integrator, a SPD 6AV UV detector and a flow-through gamma Raytest Steffi detector. The separation was carried out on a C18 column (ZORBAX 80Å, 4.6 x 150 mm). For radiotracers **[¹²²I](1), [¹²⁵I](3), [¹³¹I](1)** and **[¹²⁵I](5)** semi-preparative RP-HPLC purification was performed on a Perkin Elmer system consisting of a Flexar LC autosampler, a Series 200 pump, a Peltier column oven, a vacuum degasser, a Flexar PDA detector and a GabiStar Raytest detector. In these cases, the separation was carried out on a C18 column (SymmetryPrep C18, 7.8 × 300 mm, 7 µm, Waters). All radiotracers were shown by radio-TLC and analytical radio-RP-HPLC to be identical to the authentic nonradioactive material and to be free of significant chemical and radiochemical impurities.

### EXAMPLE 5: Radiolabelling of compound (1) with iodine-125, at low specific activity.

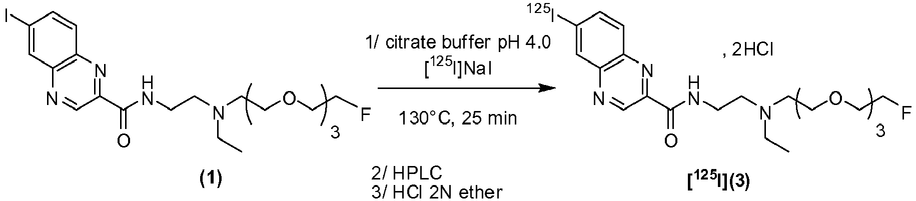

To a solution of compound **(1)** (3.2 mg) in citrate buffer pH=4 (500 µL) were added, in a sealed vial, an aqueous copper sulfate solution (0.5 mg, 100 µL) used as catalyst and [¹²⁵I]NaI (181 MBq). The reaction mixture was heated at 130 °C for 25 min. After cooling to room temperature, the residue was taken up in water (500 µL) and a 1.0 N aqueous NaOH solution (100 µL) was added. The vial cap and septum were removed. The resulting suspension was passed through an Extrelut column, the vial was rinsed twice with ethanol (150 µL), and after 10 min the column was eluted with dichloromethane (5x3 mL). The collected organic extracts were evaporated under reduced pressure, taken up with methanol (2x100 µL), and purified by semi-preparative HPLC (Waters Symmetry® Semi-Prep C18 column (300x7.8 mm, porosity 7 µm); 70% methanol (0.2% NH₄OH) in water (0.2% NH₄OH)) at a flow rate of 3 mL/min. The fraction containing the radio labelled product was collected (retention time 15.6 min), evaporated to dryness, dissolved in dichloromethane (2 mL) and treated with a 2.0 N hydrochloric acid solution in anhydrous ether (2 mL). The resulting hydrochloride solution was evaporated under reduce pressure, and the dry residue was suspended in anhydrous ether (3 mL). The solvent was then evaporated under *vacuum* for 30 min to give the radio labelled compound [¹²⁵I](3). Radiochemical yield, 50% (90 MBq); specific activity, 19.3 MBq/µmol; radiochemical purity, > 99%.

### EXAMPLE 6: Radiolabelling of compound (5) with iodine-125, at low specific activity.

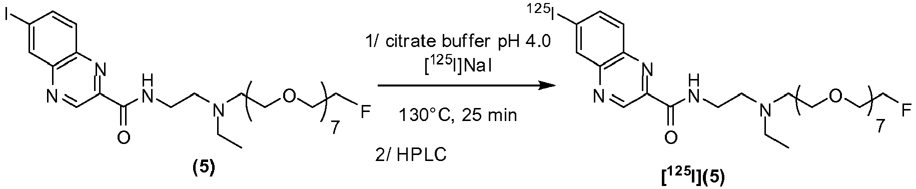

This compound was synthesized according to the procedure described for [¹²⁵I](1), except for the final hydrochloride conversion step, starting from compound (5) (4.0 mg), citrate buffer pH=4 (500 µL), an aqueous copper sulfate solution (0.5 mg, 100 µL) and [¹²⁵I]NaI (363 MBq). Reaction time: 25 min at 130 °C. Purification: semi-preparative HPLC (Waters Symmetry® Semi-Prep C18 column (300x7.8 mm, porosity 7 µm); 75% methanol (0.2% NH₄OH) in water (0.2% NH₄OH)) at a flow rate of 2.5 mL/min; retention time: 13.5 min. Radiochemical yield, 22% (78 MBq); specific activity, 15.6 MBq/µmol; radiochemical purity, > 99%.

### EXAMPLE 7: Radiolabelling of compound (7) with iodine-125, at low specific activity.

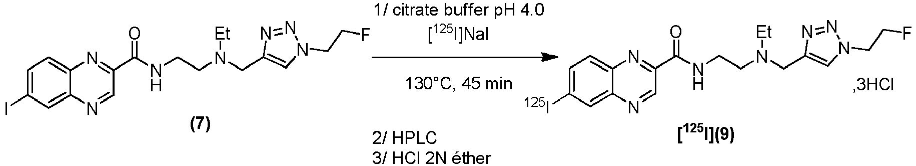

This compound was synthesized according to the procedure described for compound [¹²⁵I]**(3)**, starting from compound (7) (2.9 mg, 4.8 µmol), citrate buffer pH = 4 (500 µL), an aqueous copper sulfate solution (0.5 mg, 100 µL) and [¹²⁵I]NaI (241 MBq). Reaction time: 45 min at 130 °C. Purification by HPLC (ZORBAX 80Å C18 column, (4.6 x 150 mm); gradient time = 20 min, H₂O/MeOH (50:50 → 0:100) (NH₄OH 0.2%) at a flow rate of 1 mL/min; retention time 11.6 min. Radiochemical yield, 35%; specific activity, 34.6 MBq/µmol; radiochemical purity, 98.8%.

### EXAMPLE 8: Radiolabelling of compound (1) with iodine-131 or iodine-125, at high specific activity.

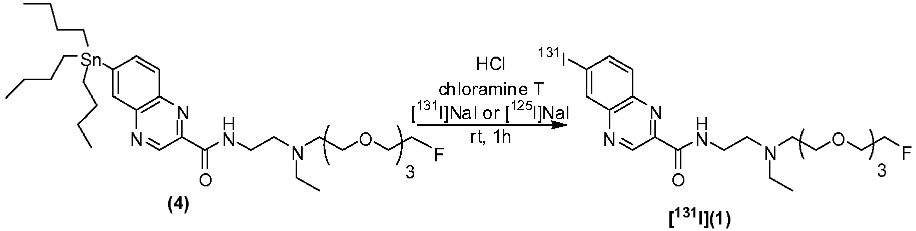

To a solution of compound **(4)** (50 µL, 1mg/mL in EtOH) were added, in a sealed vial, [¹³¹I]NaI (1538 MBq), an aqueous solution of HCl 1 N (50 µL) and an aqueous solution of chloramine T monohydrate (20 µL, 1 mg/mL). The reaction mixture was left at room temperature for 1 h (vortexed every 20 min). The mixture was quenched by addition of 1 N aqueous NaOH solution (100 µL) and aqueous sodium metabisulfite (Na₂S₂O₅) solution (10 µL, 20 mg/mL). The mixture was purified by semi-preparative HPLC (Waters Symmetry^{®} Semi-Prep C18 column (300x7.8 mm, porosity 7 µm); linear gradient 70 to 100 % methanol (0.2% NH₄OH) in water (0.2% NH₄OH) in 15 min) at a flow rate of 1.2 mL/min. The fraction containing the radio labelled product was collected (retention time 19.8 min) and evaporated to dryness to give the radiolabelled compound [¹³¹I]**1**. Radiochemical yield, 80% (1223 MBq); specific activity 99-130 GBq/µmol, octanol/water (PBS buffer) partition coefficient (logD) 0,95 ± 0,04; radiochemical purity, > 99%.

The same protocol was followed for the radiolabelling of compound (1) with iodine-125, starting from compound (**4**) (50 µL, 1mg/mL in EtOH), [¹²⁵I]NaI (304 MBq), an aqueous solution of HCl 0.5 N (50 µL) and an aqueous solution of chloramine T monohydrate (20 µL, 1 mg/mL). Radiochemical yield, 83% (251 MBq); specific activity 107-121 GBq/µmol, radiochemical purity, > 99%.

### EXAMPLE 9: Radiolabelling with fluorine-18.

### Materials for radiolabelling with fluorine-18.

No-carrier-added fluorine-18 (half-life: 109.8 minutes) was produced *via* the [¹⁸O(p, n)¹⁸F] nuclear reaction by irradiation of a 2.8 mL [¹⁸O]water target (> 97%-enriched, Bruce Technology, Chapel Hill, USA) on a CPH14 (Cyclopharma laboratories, Saint-Beauzire, France) cyclotron (14 MeV proton Beam) and the aqueous radioactive solution was then transferred to the appropriate hot cell. Typical production of [¹⁸F]fluoride at the end of bombardment for a 90 to 100 µA.h (120 min) irradiation: 185 GBq (5 Ci). Radiochemistry syntheses were performed using a mono or bi-reactor Synchrom^{®} R&D synthesis module (Raytest, Straubenhardt, Germany). Radio-TLCs were run on Merck pre-coated plates of neutral aluminium oxide 60F₂₅₄ with a mixture of dichloromethane and ethanol as solvents (98/2). Radioactive spots were detected using an AMBIS 4000 detector equipped with a computer-controlled multiwire proportional counter (Scanalytics, Rockville, USA). Semi-preparatives HPLC were conducted on a Waters Symmetry^{®} Semi-Prep C18 column (300 x 7.8 mm; porosity: 7 µm) or Waters Spherisorb^{®} Semi-Prep SiO₂ column (250x10 mm, porosity 10 µm); flow rate: 3.0 mL/min; temperature: rt; absorbance detection at λ = 254 nm. Chemical and radiochemical purities of HPLC purified and formulated compound for *in vivo* imaging were determined using analytical HPLC with the following equipment and conditions: System: a PerkinElmer Series 200 equipped with UV Diode Array Detector (DAD) and a GABI Star gamma-detector (Raytest); column: Waters Symmetry^{®} C18 (150 x 4.6 mm; porosity 5 µm) or Waters Spherisorb^{®} SiO₂ (150x4.6 mm, porosity 5 µm); flow rate: 1.0 mL/min; temperature: rt; absorbance detection at λ = 254 nm.

### Prerequisite: preparation of the Kr[¹⁸F]F-K₂₂₂-complex.

The activated K[¹⁸F]F-K₂₂₂-complex was obtained by a conventional method. Briefly, the aqueous [¹⁸F]fluoride from target was flushed through an anion exchange resin (SepPak^{®} Light Accell™ Plus QMA cartridge, Waters). The radioactivity was eluted to the reactor with 1.0 mL of a carbonate potassium (3 mg) and Kryptofix^{®} (15 mg) solution (AcN/H₂O 30/70). Solvents were evaporated under reduced pressure and under a gentle stream of helium at 110 °C for 10 min. A second azeotropic drying was repeated by addition of 1 mL of anhydrous acetonitrile.

### 9.1 Radiolabelling of compound (1) with fluorine-18.

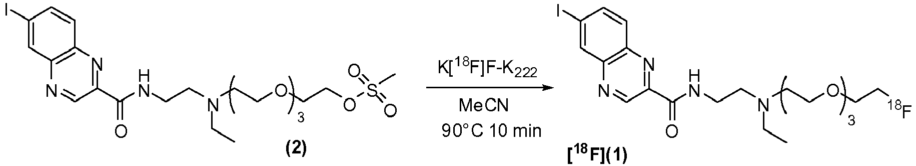

A solution of the corresponding mesylate precursor (compound (2), 13 mg) in dry acetonitrile (1 mL) was added to the dry K[¹⁸F]F-K₂₂₂-complex and heated at 90 °C for 10 min. After cooling to room temperature, the solution was diluted with 2.5 mL of HPLC eluent and purified by semi-preparative HPLC (C18 column; methanol/water/ammonium hydroxide (74.7:24.9:0.4 v/v/v; tr = 12.5 min). The collected solution was diluted with saline (20 mL) then passed through a C18 cartridge (SepPak^{®} Plus C18, Waters). The cartridge was then washed with water (5 mL) before being eluted with ethanol (900 µL) and then saline (8 mL). The solution was collected in a sterile vial (formulation yield: 90%). The radiosynthesis was accomplished within 57 min with a decay-corrected yield of 21%. Finally, [¹⁸F]**(1)** was obtained with a specific activity of 45-53 GBq/µmol and a radiochemical purity of > 99%, as confirmed by analytical HPLC (C18 column; methanol/water/ammonium hydroxide v/v/v; 59.9:39.9:0.2 to 89.8:10:0.2 linear gradient in 10 min then 59.9:39.9:0.2 isocratic during 7 min; 0.9 mL/min; tr = 12.51 min). Color spot test showed no detection of K₂₂₂ in the final injectable [¹⁸F]**(1)** solution. Radiotracer was radiochemically stable at room temperature for at least 24 h after preparation. The octanol/water (PBS buffer) partition coefficient (logD) for [¹⁸F]**(1)** was 1.52±0.03.

### 9.2 Radiolabelling of compound (5) with fluorine-18.

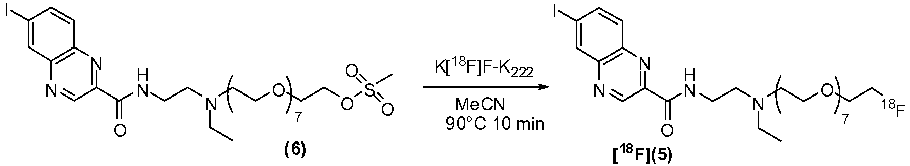

A solution of corresponding mesylate precursor (compound (6), 14 mg) in dry acetonitrile (1 mL) was added to the dry K[¹⁸F]F-K₂₂₂-complex then heated at 90 °C for 10 min. After cooling to room temperature, the solution was diluted with 2.5 mL of HPLC eluent and purified by semi-preparative HPLC (SiO₂ column; dichloromethane/ethanol/ammonium hydroxide 91.8:8:0.2 v/v/v; tr = 12.1 min). Formulation: The solvent was evaporated under vacuum and the formulation was achieved in ethanol/saline 1/9 (v/v) (formulation yield: 93%). The radiosynthesis was accomplished within 65 min with a decay-corrected yield of 34%. Finally, [¹⁸F]**(5)** was obtained with a specific activity of 81-139 GBq/µmol and a radiochemical purity of > 99%, as confirmed by analytical HPLC (Waters Spherisorb^{®} SiO₂; 10% EtOH (0.2% NH₄OH) in CH₂Cl₂ (0.2% NH₄OH), *t*_{R}=9.1 min). Color spot test showed no detection of K₂₂₂ in the final injectable [¹⁸F]**(5)** solution. Radiotracer was radiochemically stable at room temperature for at least 6 h after preparation. The octanol/water (PBS buffer) partition coefficient (logD) for [¹⁸F]**(5)** was 0.95±0.02.

### 9.3 Radiolabelling of compound (7) with fluorine-18.

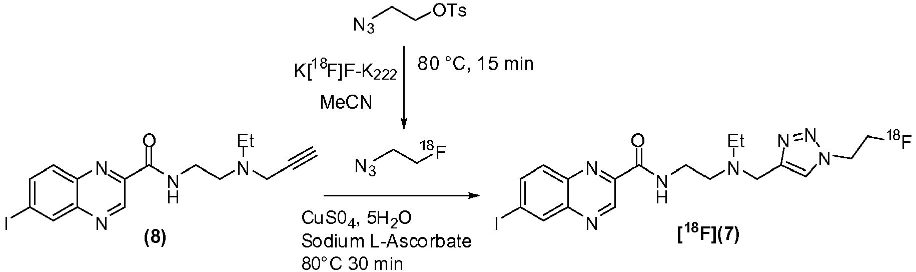

A solution of 2-azidoethyl-4-toluenesulfonate (5 µL, 6 mg) in dry acetonitrile (1 mL) was added to the dry K[¹⁸F]F-K₂₂₂-complex and heated at 80 °C for 15 min. After cooling to room temperature, the solution was distilled at 110 °C under a gentle flow of helium (20 mL/min) into the second reaction vial containing a solution of copper sulfate pentahydrate (4.89 mg) in sodium acetate buffer (250 mM, 50 µL) and compound (8) (4.5 mg) in dry DMF (200 µL). Second reactor temperature was maintained at 20 °C during the complete distillation process (5 min), leading a yield of transfer of 82%. A solution of sodium L-ascorbate (4.4 mg) in sodium acetate buffer (250 mM, 550 µL) was added to the mixture and heated at 80 °C for 30 min. After cooling, the solution was diluted with 2 mL of HPLC eluent and purified by semi-preparative HPLC (Waters Symmetry^{®} Semi-Prep C18 column (300x7.8 mm, porosity 7 µm); 70% methanol (0.4% NH₄OH) in water (0.4% NH₄OH)). Formulation: The collected solution was diluted in 20 mL of saline then passed through a C18 cartridge (SepPak^{®} C18, Waters). The cartridge was then washed with water (5 mL) before being eluted with 300 µL of ethanol followed by 2.7 mL of physiological saline.
The radiosynthesis was accomplished within 85 min with a decay-corrected yield of 36%. Finally, [¹⁸F]**(7)** was obtained with a radiochemical purity of >99%, as confirmed by analytical HPLC (Waters Symmetry^{®} C18; Gradient elution: 60% to 90% MeOH (0.4%NH₄OH) in water (10 min), then 60% for 7 min, *t*_{R}=9.63 min). Color spot test showed no detection of K₂₂₂ in the final injectable [¹⁸F]**7** solution. Radiotracer was radiochemically stable at room temperature for at least 6 h after preparation. The octanol/water (PBS buffer) partition coefficient (logD) for [¹⁸F]**(7)** was 2.22±0.03.

Some of the compounds of the invention have been subject of pharmacological tests reported below.

### EXAMPLE 10: PHARMACOLOGY EXPERIMENTATION

### 10.1 Assessment of [¹⁸F]1, [¹⁸F]5 and [¹⁸F]7 biodistribution in primary model of melanoma

### 10.1.1 Experimental protocols.

All our studies were performed in accordance with French and European Economic Community (86-609, EEC) guidelines for care of laboratory animals. Protocols were performed under the authorization of the French Direction des Services Vétérinaires (authorization no. CE 86-12). Male C57BL/6J mice were originating from Charles River laboratories and maintained at 21 °C with a 12 h/12 h light/dark cycle, fed with a breeding diet (diet A04 from Safe, Villemoisson, France) and received water *ad libitum*. For transplantation B16BL6 cell suspension were grown in a monolayer culture to confluence, after which cells were trypsinized, washed with phosphate buffer saline (PBS) and they were resuspended in PBS. Mice were anesthetized by isoflurane (4% for induction and 2±0.5% for maintaining in oxygen flux) inhalation. The primary tumour model was induced by subcutaneous injection of 3x10⁵ melanoma B16B16 cells in 0.1 mL on the right shoulder of mice.

Whole-body PET scans were acquired 1 h after bolus injection of 9±12 MBq of the appropriate [¹⁸F]-compound in a volume of 0.15 ml via the tail vein. Mice were anesthetized by intraperitoneal (i.p.) administration (200 µL/mouse) of a ketamine-xylazine mixture in saline: ketamine (100 mg/kg, Imalgene™, Rhone Mérieux, Lyon, France) and xylazine (10 mg/kg, Rompun™, Bayer, France) before radiotracer injection and imaged by using a small-animal PET device, eXplore Vista microPET instrument (GE Healthcare). PET images were acquired by using static mode during 20 min. The acquired data were reconstructed using the 2-Dimensionnal ordered-subsets expectation maximization (OSEM) algorithm and corrected from decay, scatter and randoms. No corrections were applied for partial volume or attenuation.

Regional tracer uptake was quantified by standard region-of-interest (ROI) analysis with eXplore Vista software package. A manual 2D-ROI was drawn around the tumour in each slice where tumour was visible, and on several subsequent coronal planes containing muscle tissue at the forelimb. Results are expressed by percentage of injected dose per gram of tissue (%ID/g).

*Ex vivo* biodistribution studies: An ex vivo biodistribution study was performed for better comparison of [¹⁸F]**(1)** and [¹⁸F]**(5)**. Experimentations were undertaken at day-10 or day-14 after cell inoculation (n=3-6/group). Animals were sacrificed by CO₂ asphyxiation 1 h after i.v. injection of [¹⁸F]-labelled compound (9-12 MBq). Tumours and major organs and tissues were promptly excised, harvested, weighted and their radioactivity counted using a calibrated γ-counter with a 400 to 600 keV energy window. After radioactive decay correction, results were expressed as percentage of injected dose per gram of tissue (%ID/g).

[¹⁸F]**1** stability study on *ex vivo* extracts: Six primary melanoma bearing mice were intravenously injected with 18 MBq of [¹⁸F]**(1)** and sacrificed 30 min, 1 h and 2 h after tracer injection. To determine the metabolic stability of [¹⁸F]**(1)**, blood, tumour, eyes, liver and urine samples were collected. Blood was immediately centrifuged for 5 min at 3,000 rpm and plasma directly investigated by analytical HPLC. Other organs were pooled and homogenized using GentleMACS™ dissociator (Miltenyi Biotec, Bergisch Gladbach, Germany), suspended in 5 mL of methanol/NH₄OH 99.8:0.2 and centrifuged for 5 min at 3,000 rpm. The extraction efficiency was determined by measuring the radioactivity of supernatants and precipitates. Supernatants were passed through a 0.22 µm Millipore filter and then concentrated to a volume between 200 and 500 µL before injection (duplicate) onto the analytical HPLC (Waters Symmetry^{®} C18; Gradient elution: 60% to 90% MeOH (0.4% NH₄OH) in water (0.4% NH₄OH) (10 min), then 60% for 7 min).

### 10.1.2 Biodistribution results

PET imaging was performed 1 h post injection of tracers in B16BL6 tumour bearing mice at day-15 after tumours cell inoculation.

PET imaging with [¹⁸F]**(1)**: Results showed that [¹⁸F]**(1)** was rapidly taken up and accumulated in the tumour with an uptake of 14.33±2.11% ID/g (n=8). High tracer uptake was also observed in other melanotic tissue such as eyes (10.50 ± 2.95% ID/g; n=8) and pigmented spleen (7.38±0.73% ID/g; n=4) in accordance to melanin specificity of this radiotracer. A significant radioactive signal was also observed in bladder indicating a rapid elimination by urinary excretion and also in intestine (4.07±1.39% ID/g; n=6) indicating a partial fecal elimination. By contrast, activity in non-target tissues such as muscles, displayed a fast washout at 1 h p.i. (1.37 ± 0.37% ID/g; n=8).

PET imaging with [¹⁸F]**(5)**: Results showed that [¹⁸F]**(5)** tumour uptake was markedly lower than that of [¹⁸F]**(1)** (6.63±1.64% ID/g (n=3)). High tracer uptake was also observed in other melanotic tissue such as eyes (5.53±1.35% ID/g; n=3) and in the gastrointestinal tract. By contrast, activity in non-target tissues such muscles, displayed a fast washout at 1 h p.i. (1.01± 0.09% ID/g; n=3).

PET imaging with [¹⁸F]**(7)**: Results showed that [¹⁸F]**(7)** uptake was of 14.55% ID/g (n=1) in the tumour and 0.84% ID/g (n=1) in the muscle. As for [¹⁸F]**(1)**, a fast washout from non target organs was also observed.

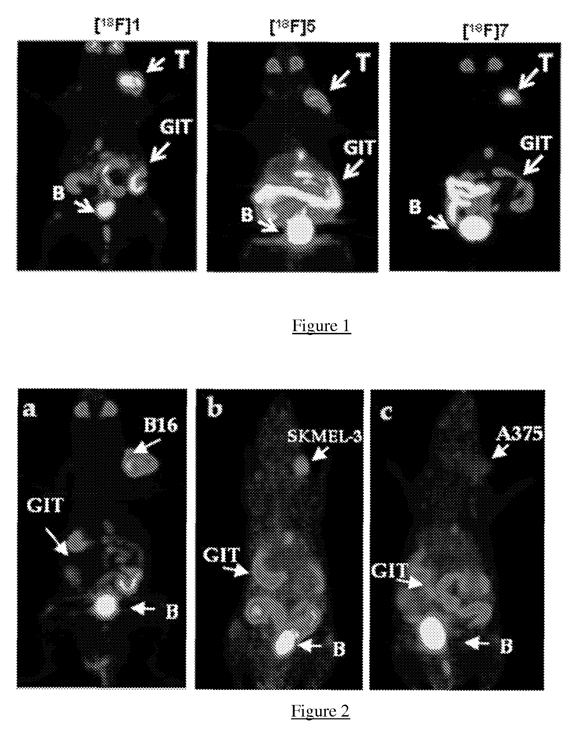

Figure 1 displays PET images of three B16BL6 tumour bearing mice performed at day 15, 1h after injection of [¹⁸F]**(1)** (a), [¹⁸F]**(5)** (b) or [¹⁸F]**(7)** (c). Tumour (T), gastrointestinal tract (GIT), Stomach (S), and bladder (B) are indicated.

Compounds [¹⁸F]**(1)** and [¹⁸F]**(5)** were studied in *ex vivo* biodistribution experiments by direct-sampling tumours and tissues of interest at 1 h, 2 h and 3 h post tracer injection. Uptakes in pigmented tissues were similar to those obtained by PET imaging at 1 h p.i. and remained high and stable until 3 h p.i. for both molecules (i.e. tumoural uptake at 3 h p.i. was 11.55±1.24% ID/g for [¹⁸F]**(1)** and 6.76±1.35% ID/g for [¹⁸F]**(5)**). Furthermore, a better tumoural uptake was observed for [¹⁸F]**(1)** compared to [¹⁸F]**(5)**, at all time points. Very low tracers accumulations in muscle and blood led to high tumour-to-muscle and tumour-to-blood ratios reaching values of 14.46±3.06 and 14.43±1.75 respectively for [¹⁸F]**(1)** and values of 6.64±2.69 and 5.27±0.20 respectively for [¹⁸F]**(5)** at 3 h p.i. Low bones uptakes were observed at 1 h p.i. (1.40±0.10% ID/g for [¹⁸F]**(1)** and 1.82±0.55% ID/g for [¹⁸F]**(5)**). Partial *in vivo* defluorinations of [¹⁸F]**(1)** and [¹⁸F]**(5)** occurred at 3 h p.i., which were not correlated with *in vitro* stability in saline (RCP >99% for at least 6 h).

**Table 1 : Ex vivo biodistribution of [¹⁸F](1) and [¹⁸F](5) in C57BL/6J mice bearing B16 murine melanoma tumours at 1 h, 2 h, and 3 h p.i. Data are expressed as percentage of injected dose per gram of tissue (%ID/g)±SD (n=5-6).**

| | **[¹⁸F](1)** | | | **[¹⁸F](5)** | | |
|---|---|---|---|---|---|---|
| **%ID/g** | **1 h (n=5)** | **2 h (n=6)** | **3 h (n=5)** | **1 h (n=6)** | **2 h (n=5)** | **3 h (n=6)** |
| **Tumour** | 14.05 ± 1.42 | 13.07 ± 1.22 | 11.55 ± 1.24 | 9.81 ± 2.96 | 5.81 ± 1.37 | 6.76 ± 1.35 |
| **Muscle** | 1.17 ± 0.12 | 0.92 ± 0.15 | 0.81 ± 0.10 | 1.07 ± 0.35 | 1.04 ± 0.24 | 1.11 ± 0.34 |
| **Eyes** | 18.36 ± 4.74 | 18.17 ± 3.15 | 17.96 ± 2.73 | 12.24 ± 4.39 | 12.10 ± 1.85 | 13.43 ± 2.17 |
| **Bones** | 1.40 ± 0.10 | 1.96 ± 0.51 | 3.27 ± 0.70 | 1.82 ± 0.55 | 2.13 ± 0.19 | 3.61 ± 1.12 |
| **Blood** | 1.42 ± 0.32 | 1.12 ± 0.24 | 0.80 ± 0.07 | 1.15 ± 0.19 | 1.37 ± 0.30 | 0.99 ± 0.20 |
| **Skin** | 1.44 ± 0.19 | 1.51 ± 0.53 | 1.05 ± 0.12 | 1.22 ± 0.22 | 0.94 ± 0.51 | 1.47 ± 0.49 |
| **Liver** | 2.56 ± 0.22 | 1.88 ± 0.34 | 1.99 ± 0.45 | 2.02 ± 0.19 | 2.31 ± 0.64 | 2.60 ± 1.37 |
| **Stomach** | 4.41 ± 1.36 | 2.48 ± 0.88 | 2.81 ± 2.23 | 5.87 ± 3.32 | 7.35 ± 4.79 | 5.39 ± 3.13 |
| **Small intestine** | 7.58 ± 1.80 | 4.28 ± 1.18 | 3.91 ± 1.05 | 16.68 ± 7.93 | 19.51 ± 5.43 | 18.62 ± 13.65 |
| **Ceacum** | 7.27 ± 0.62 | 8.94 ± 1.78 | 10.79 ± 1.54 | 6.37 ± 4.84 | 12.10 ± 10.31 | 29.31 ± 17.94 |
| **Colon+faeces** | 7.17 ± 2.93 | 6.05 ± 1.72 | 8.09 ± 5.04 | 7.39 ± 1.36 | 8.14 ± 1.78 | 12.63 ± 7.83 |
| **Kidney** | 3.46 ± 0.53 | 2.18 ± 0.43 | 2.19 ± 0.36 | 3.60 ± 1.74 | 3.52 ± 1.02 | 2.60 ± 1.69 |
| **Spleen** | 3.72 ± 1.49 | 2.88 ± 2.11 | 1.87 ± 0.38 | 2.35 ± 0.91 | 2.38 ± 0.87 | 2.35 ± 0.98 |
| **Pancreas** | 2.09 ± 0.42 | 1.33 ± 0.28 | 1.33 ± 0.35 | 2.63 ± 1.33 | 1.56 ± 0.38 | 1.81 ± 0.66 |
| **Lung** | 1.86 ± 0.15 | 1.65 ± 0.11 | 1.60 ± 0.09 | 1.75 ± 0.18 | 1.72 ± 0.72 | 2.37 ± 0.67 |
| **Heart** | 2.72 ± 0.18 | 2.14 ± 0.33 | 2.32 ± 0.50 | 1.58 ± 0.24 | 1.70 ± 0.24 | 3.00 ± 1.12 |
| **Brain** | 1.82 ± 0.08 | 1.39 ± 0.25 | 1.37 ± 0.35 | 1.03 ± 0.18 | 1.03 ± 0.10 | 1.72 ± 0.67 |
| **Ratio** | | | | | | |
| **TMR^{a}** | 12.12 ± 2.29 | 14.46 ± 2.62 | 14.46 ± 3.06 | 9.48 ± 2.41 | 5.75 ± 1.45 | 6.64 ± 2.69 |
| **TBR^{b}** | 10.24 ± 2.83 | 12.19 ± 3.32 | 14.43 ± 1.75 | 8.29 ± 2.87 | 4.01 ± 1.12 | 5.27 ± 0.20 |
| **TBoR^{c}** | 10.11 ± 1.87 | 7.13 ± 2.21 | 3.70 ± 1.17 | 5.35 ± 0.82 | 2.75 ± 0.80 | 2.16 ± 1.15 |

| | | | | | | |
|---|---|---|---|---|---|---|
| *^{a}*Tumour-to-Muscle Ratio ; ^{b}Tumour-to-Blood Ratio; ^{c}Tumour-to-Bone Ratio | | | | | | |

**Table 2: [¹⁸F](1) stability study on ex vivo extracts.**

| % unchanged [¹⁸F]**(1)** | | 30 min | 1h | 2h |
|---|---|---|---|---|
| | Blood | 15.2 | n.d. | n.d. |
| | Tumour | n.d. | >99 | 100 |
| Black | Eyes | n.d. | 100 | >99 |
| | Liver | n.d. | <10 | <20 |
| | Urine | n.d. | 0.1 | 0.0 |
| | Blood | 3.0 | n.d. | n.d. |
| Nude | Tumour | n.d. | >99 | >99 |
| | Liver | n.d. | n.d. | <10 |
| | Urine | n.d. | 2.7 | 1.4 |

### 10.2. Assessment of [¹⁸F](1), biodistribution in primary model of human melanoma models

### 10.2.1. Experimental protocol.

Protocols were performed under the authorization of the French Direction des Services Vétérinaires (authorization no. CE 86-12). Female Swiss nudes mice (6 weeks old) were originating from Charles River laboratories and maintained at 21 °C with a 12 h/12 h light/dark cycle, fed with a breeding diet (diet A04 from Safe, Villemoisson, France) and received water *ad libitum*. For transplantation cell suspension were grown in a monolayer culture to confluence, after which cells were trypsinized, washed with phosphate buffer saline (PBS) and they were resuspended in PBS. Mice were anesthetized by isoflurane (4% for induction and 2±0.5% for maintaining in oxygen flux) inhalation. The primary tumour model was induced by subcutaneous injection of 3x10⁶ melanoma SKMEL-3 (pigmented cell line) or A375 (non-pigmented cell ligne) cells in 0.1 mL on the right shoulder of mice.

Whole-body PET scans were acquired 1 h after bolus injection of ∼10 MBq of [¹⁸F]**(1)** in a volume of 0.15 ml via the tail vein. Mice were anesthetized by intraperitoneal (i.p.) administration (200 µL/mouse) of a ketamine-xylazine mixture in saline: ketamine (100 mg/kg, Imalgene™, Rhone Mérieux, Lyon, France) and xylazine (10 mg/kg, Rompun™, Bayer, France) before radiotracer injection and imaged by using a small-animal PET device, eXplore Vista microPET instrument (GE Healthcare). PET images were acquired by using static mode during 20 min. The acquired data were reconstructed using the 2-Dimensionnal ordered-subsets expectation maximization (OSEM) algorithm and corrected from decay, scatter and randoms. No corrections were applied for partial volume or attenuation.

Regional tracer uptake was quantified by standard region-of-interest (ROI) analysis with eXplore Vista software package. A manual 2D-ROI was drawn around the tumour in each slice where tumour was visible, and on several subsequent coronal planes containing muscle tissue at the forelimb. Results are expressed by percentage of injected dose per gram of tissue (%ID/g).

### 10.2.2. Biodistribution results

PET imaging was performed 1 h post injection of tracers in SKMEL-3 and A375 tumour bearing mice at day-27 and day-23 post inoculation respectively.

Results showed that [¹⁸F]**(1)** was rapidly taken up and accumulated in the tumour with an uptake of 4.65±1.07% ID/g (n=4) SKMEL-3 tumours while low tracer accumulation was observed in unpigmented A375 tumours (1.87±0.34% ID/g (n=4)) in accordance to melanin specificity of this radiotracer.

A significant radioactive signal was also observed in bladder and intestine indicating a rapid elimination by both urinary excretion and fecal elimination. By contrast, activity in non-target tissues such as muscles, displayed a fast washout at 1 h p.i. (1.34±0.42% ID/g for SKMEL-3 tumour bearing mice and 1.14±0.28% ID/g for A375 tumour bearing mice).

Figure 2 displays PET images of B16BL6 tumour bearing mice (a), SKMEL-3 tumour bearing mice (b) and A375 tumour bearing mice (c) performed at day-15, day-27 and day-22 respectively, 1 h after injection of [¹⁸F]**(1)**. Tumour, gastrointestinal tract (GIT), and bladder (B) are indicated.

### 10.3. Assessment of [¹²⁵I]1, [¹²⁵I]5 and [¹²⁵I]7 biodistribution in primary model of melanoma

### 10.3.1 Experimental protocol.

Protocols were performed under the authorization of the French Direction des Services Vétérinaires (authorization no. C63-113-10) and conducted under the supervision of authorized investigators in accordance with the institution's recommendations for the use of laboratory animals. Animals were handled and cared in accordance with the guidelines for the Care and Use of Laboratory Animals (National Research Council, 1996) and European Directive 86/609/EEC.

C57BL/6J male mice (6-8 weeks old) were obtained from Charles River (l'Arbresle, France) and the B16F0 syngenic melanoma from ATCC (no. CRL-6322). Primary melanoma model was induced by subcutaneous injection of B16F0 murine melanoma cells in C57BL/6J mice. B16F0 melanoma cell cultures were maintained as monolayers in Dulbecco's Modified Eagle's Medium (DMEM)/Glutamax (Invitrogen, Cergy Pontoise, France) supplemented with 10% fetal calf serum (Sigma, Saint Quentin Fallavier, France), 1% vitamins (Invitrogen, Cergy-Pontoise, France), 1 mM sodium pyruvate (Invitrogen), 1% non essential amino acids (Invitrogen), and 4 µg/mL of gentamycin base (Invitrogen). Cells were grown at 37 °C in a humidified incubator containing 5% CO₂. For transplantation, cells in exponential growth phase were trypsinized, washed with phosphate buffer saline (PBS), and resuspended in PBS. C57BL/6J mice anesthetized by isoflurane (2%) inhalation were inoculated with 3x10⁵ melanoma B16F0 cells in 0.1 mL of PBS by subcutaneous injection on the right flank.

### 10.3.2. In vivo scintigraphic imaging

*In vivo* scintigraphic imaging protocol was submitted and approved by the french regional ethical animal use committee (CEMEA, Auvergne, authorization no. CE18-09, 2010). Scintigraphic imaging of radioiodinated compounds in mice was performed using a gamma camera dedicated to small animal imaging (γIMAGER, Biospace Mesures, Paris, France). This camera consists of a R 3292 Hamamatsu position-sensitive photomultiplier having a continuous 4 mm thick x 120 mm diameter CsI (Na) crystal leading to a 10 cm field of view. For ¹²⁵I imaging, the camera was equipped with parallel-hole collimator 1.8/0.2/20 (hole diameter/septum thickness/height in mm). All the acquisitions were performed with a 15% window centered on the 35 keV peak of ¹²⁵I.

On day 14 after tumour implantation, each [¹²⁵I]-labelled compound was administered intravenously *via* the tail vein with 3.7 MBq/mouse (range 3 - 8 animals/compounds). The injected activity to each mouse was also determined from scintigraphic imaging of the syringe before and after the injection of the radiotracer. At different times after administration (1 h, 3 h, 6 h, 24 h, 72 h, 5 d, 7 d, 10 d and 14 d), mice were anaesthetized by *i.p.* administration (200 µL/mouse) of a ketamine-xylazine mixture in saline: ketamine (100 mg/kg, Imalgene, Rhone Mérieux, Lyon, France) and xylazine (10 mg/kg, Rompun, Bayer, France). A 10 min duration image was acquired on anesthetized mice placed in prone position over the collimator of the camera. Reproducibility in animal positioning for the serial images was achieved using a graduated reference grid.

Quantitative analysis of scintigraphic scans was performed using the GAMMAVISION+ software (Biospace Mesures, Paris, France). Regions of interest (ROIs) were manually drawn around the tumour, the whole body area, the bladder, the eyes, the submaxillary and thyroid glands and the controlateral muscle which was chosen as background. For each ROI, total activity, min and max pixel values, ROI size (in mm²), average activity per mm² and standard deviation were obtained. These values were normalized to the injected activity. For the tumour, the activity was also normalized to the tumour weight as previously described (Miot-Noirault, Cancer biotherapy & radiopharmaceuticals, 24 (2009) 629-636.) and expressed as percentage of injected dose per gram of tumour (%ID/g).

**Table 3: Radioactivity uptake in melanoma B16F0 tumours at different time points after i.v. injection of radiotracers labelled with iodine-125 in C57BL/6J mice. Radioactive concentration values were calculated from planar scintigraphic images.**

| %ID/g (tumour) | 1 h | 3 h | 6 h | 24 h | 72 h | 5 d | 7 d | 10 d | 14 d |
|---|---|---|---|---|---|---|---|---|---|
| [¹²⁵I]**5** | 6.8 ± 1.8 n = 4 | 7.8 ± 1.5 n = 4 | 7.2 ± 1.1 n = 4 | 6.8 ± 1.7 n = 4 | 3.8 ± 0.7 n = 4 | 2.7 ± 0.3 n = 4 | 1.5 ± 0.2 n = 3 | 0.8 ± 0.3 n = 3 | 0.3 ± 0.01 n = 2 |
| [¹²⁵I]**7** | 13.0 ± 1.5 n = 3 | 14.5 ± 0.1 n = 3 | 15.9 ± 1.7 n = 3 | 15.6 ± 3.4 n = 3 | 11.0 ± 0.7 n = 3 | 8.6 ± 0.4 n = 3 | 5.3 ± 0.8 n = 3 | 3.7 ± 0.3 n = 3 | 1.8 ± 0.4 n = 3 |
| [1²⁵I]**3** | 11.7 ± 2.2 n = 8 | 12.1 ± 1.7 n = 7 | 12.1 ± 1.9 n = 7 | 12.4 ± 2.3 n = 7 | 8.6 ± 3.6 n = 7 | 5.8 ± 2.4 n = 7 | 2.7 ± 0.8 n = 7 | 1.3 ± 0.4 n = 7 | 0.6 ± 0.3 n = 7 |

### 10.3.3. Ex vivo biodistribution study for compound [¹²⁵I](1)

Twenty four B16BL6 primary melanoma bearing mice were intravenously injected with 2.2 MBq of [¹²⁵I]**1** and sacrificed 1 h, 3 h, 6 h, 24 h, 72 h and 10 days after tracer injection (n=4/time point). Tumours and major organs and tissues were promptly excised, harvested, weighted and their radioactivity counted using a calibrated γ-counter with a 400 to 600 keV energy window. After radioactive decay correction, results were expressed as percentage of injected dose per gram of tissue (% ID/g).

**Table 4 : Ex vivo biodistribution of [¹²⁵I]1 in C57BL/6J mice bearing B16 murine melanoma tumours at 1 h, 3 h, 6 h, 24 h, 72 h and 10 days p.i. Data are expressed as percentage of injected dose per gram of tissue (%ID/g)±SD (n=4/time point).**

| **Organs** | **1h** | **3h** | **6h** | **24h** | **72h** | **10d** |
|---|---|---|---|---|---|---|
| Blood | 0.97±0.07 | 0.82±0.05 | 0.62±0.20 | 0.06±0.01 | 0.03±0.01 | 0.00±0.00 |
| Tumour | 11.99±3.52 | 17.69±1.10 | 12.04±4.39 | 10.47±3.22 | 8.74±0.38 | 1.20±0.54 |
| Eyes | 15.01±4.84 | 15.48±2.57 | 17.40±2.21 | 20.30±6.08 | 14.87±1.18 | 9.97±2.24 |
| Liver | 6.33±1.69 | 3.37±0.14 | 2.02±0.64 | 0.46±0.10 | 0.08±0.00 | 0.01±0.00 |
| Kidney | 4.75±0.71 | 3.14±1.27 | 1.56±0.82 | 0.15±0.02 | 0.24±0.04 | 0.04±0.03 |
| Skin | 1.04±0.06 | 1.34±0.72 | 0.62±0.31 | 0.17±0.00 | 0.50±0.26 | 0.05±0.06 |
| Adipose | 0.61±0.01 | 0.36±0.05 | 0.19±0.06 | 0.028±0.00 | 0.02±0.00 | 0.01±0.01 |
| Muscle | 0.55±0.16 | 0.30±0.04 | 0.17±0.07 | 0.02±0.00 | 0.02±0.00 | 0.01±0.06 |
| Bones | 0.79±0.09 | 0.48±0.03 | 0.30±0.10 | 0.03±0.00 | 0.02±0.01 | 0.01±0.01 |
| Colon | 4.51±4.40 | 21.19±18.02 | 15.92±9.97 | 0.69±0.23 | 0.25±0.10 | 0.02±0.01 |
| Ceacum | 4.97±3.01 | 25.73±7.88 | 23.31±16.47 | 0.41±0.13 | 0.33±0.29 | 0.02±0.02 |
| Small intestine | 16.68±10.70 | 4.16±0.70 | 2.23±1.35 | 0.12±0.03 | 0.07±0.03 | 0.01±0.00 |
| Stomach | 9.2±1.49 | 4.53±0.94 | 7.39±4.08 | 0.54±0.25 | 0.16±0.05 | 0.03±0.01 |
| Spleen | 2.14±0.20 | 2.37±2.58 | 6.45±8.15 | 0.06±0.01 | 0.52±0.96 | 0.23±0.41 |
| Pancreas | 2.96±0.15 | 1.48±0.07 | 0.65±0.18 | 0.05±0.01 | 0.03±0.01 | 0.02±0.03 |
| Lung | 3.12±0.42 | 2.14±0.27 | 1.04±0.37 | 0.08±0.01 | 0.05±0.01 | 0.01±0.01 |
| Heart | 1.17±0.07 | 0.77±0.05 | 0.46±0.17 | 0.16±0.06 | 0.08±0.02 | 0.03±0.01 |
| Brain | 0.27±0.03 | 0.15±0.01 | 0.08±0.03 | 0.01±0.01 | 0.01±0.00 | 0.00±0.00 |
| Testes | 0.68±0.19 | 0.54±0.03 | 0.36±0.08 | 0.03±0.01 | 0.02±0.00 | 0.00±0.00 |

### 10.3.4 Metabolism of compound [¹²⁵I](1)

24 primary melanoma bearing mice were intravenously injected with 2.2 MBq of [¹²⁵I]**(1)** and sacrificed 1 h, 3 h, 6 h, 24 h, 72 h and 10 days after tracer injection. To determine the metabolic stability of [¹²⁵I]**(1)**, blood, tumour, eyes, liver, kidney and urine samples were collected at each time point (n=3). Faeces were collected between 0-24 h and 24-48 h. Blood was immediately diluted in 5 mL of methanol/NH₄OH 99.8:0.2 and centrifuged for 5 min at 3,000 rpm. Tumour, eyes, liver, kidneys and faeces were suspended in 5 mL of methanol/NH₄OH 99.8:0.2, homogenized using GentleMACS™ dissociator (Miltenyi Biotec, Bergisch Gladbach, Germany) and centrifuged for 5 min at 3,000 rpm (repeated 2 times). Urines were analysed without any treatment. The extraction efficiency was determined by measuring the radioactivity of supernatants and precipitates. Supernatants were passed through a 0.22 µm Millipore filter and then concentrated to a volume between 200 and 500 µL before injection (duplicate) onto the analytical HPLC (C18 column; solvent A : H₂O (0.2% NH₄OH); solvent B : MeOH (0.2% NH₄OH); Gradient elution: 0-5 min : 90 to 70% of A (Q=0.5 mL.min⁻¹); 5-30 min : 70 to 20% of A (Q=0.9 mL.min⁻¹); 30-32 min : 10% of A (Q=0.9 mL.min⁻¹)).

The *in vivo* metabolic pathway is presented below.

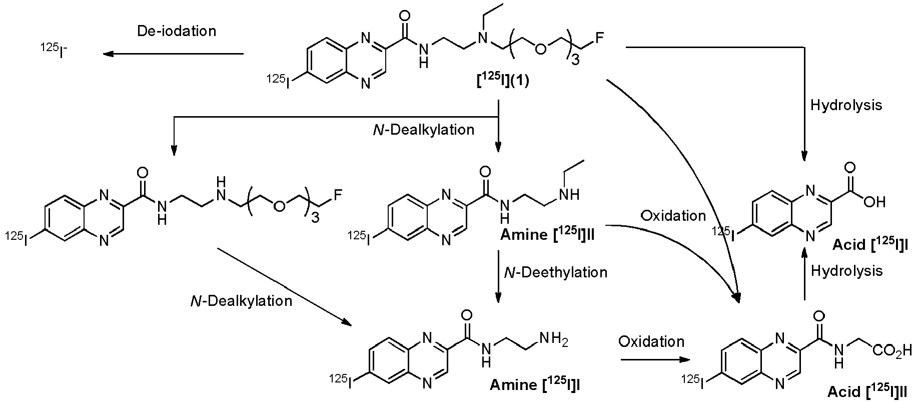

Unchanged [¹²⁵I]**(1)**, [¹²⁵I]acids I and II, [¹²⁵I]amines I and II and [¹²⁵I]I⁻ were identified and quantified as showed in the following table.

**Table 5**

| % of radioactivity | 1 h | 3h | 6h | 24 h | 72 h | 10 d |
|---|---|---|---|---|---|---|
| Eyes | | | | | | |
| Unchanged [¹²⁵I]**(1)** | 90.5±0.7 | 94.5±2.1 | 96.0±5.7 | 90.0±2.8 | 96.5±4.9 | 100.0±0.0 |
| [¹²⁵I]I⁻ | 9.5±0.7 | 5.5±2.1 | 4.0±5.7 | 0.0±0.0 | 0.0±0.0 | 0.0±0.0 |
| Tumour | | | | | | |
| Unchanged [¹²⁵I]**(1)** | 87.0±1.4 | 81.0±1.4 | 77.0±0.0 | 92.5±9.2 | 91.5±3.5 | 100.0±0.0 |
| [¹²⁵I]I⁻ | 13.0±1.4 | 6.5±0.7 | 9.0±0.0 | 0.0±0.0 | 0.0±0.0 | 0.0±0.0 |
| Liver | | | | | | |
| Unchanged [¹²⁵I]**(1)** | 27.5±0.7 | 24.0±4.2 | 8.5±0.7 | 0.0±0.0 | n.d. | n.d. |
| Amine [¹²⁵I]I | 26.0±0.0 | 45.5±2.1 | 55.0±2.8 | 70.5±0.7 | n.d. | n.d. |
| [¹²⁵I]I⁻ | 20.0±1.4 | 21.5±4.9 | 29.5±0.7 | 29.5±0.7 | n.d. | n.d. |
| Kidney | | | | | | |
| Unchanged [¹²⁵I]**(1)** | 12.5±2.1 | 6.1±2.9 | 0.0±0.0 | n.d. | n.d. | n.d. |
| Amine [¹²⁵I]I | 18.5±0.7 | 11.7±0.7 | 0.0±0.0 | n.d. | n.d. | n.d. |
| Acid [¹²⁵I]I | 29.0±1.4 | 34.2±0.7 | 43.0±1.4 | n.d. | n.d. | n.d. |
| [¹²⁵I]I⁻ | 20.5±0.7 | 37.2±2.2 | 57.0±1.4 | n.d. | n.d. | n.d. |
| Blood | | | | | | |
| Unchanged [¹²⁵I]**(1)** | 2.0±2.8 | 0.0±0.0 | 3.5±4.9 | n.d. | n.d. | n.d. |
| Acid [¹²⁵I]I | 21.5±2.1 | 11.5±0.7 | 11.0±0.0 | n.d. | n.d. | n.d. |
| [¹²⁵I]I⁻ | 76.5±0.7 | 88.5±0.7 | 85.5±4.9 | n.d. | n.d. | n.d. |
| Urine | | | | | | |
| Unchanged [¹²⁵I]**(1)** | 12.6±0.5 | 0.0±0.0 | 0.0±0.0 | 0.0±0.0 | 0.0±0.0 | n.d. |
| Amine [¹²⁵I]I | 6.6±0.6 | 13.0±1.4 | 3.5±4.9 | 0.0±0.0 | 0.0±0.0 | n.d. |
| Acid [¹²⁵I]I | 17.2±3.1 | 20.0±1.4 | 16.0±1.4 | 3.5±0.7 | 0.0±0.0 | n.d. |
| Acid [¹²⁵I]II | 30.3±2.4 | 24.0±0.0 | 21.0±0.0 | 6.5±0.7 | 0.0±0.0 | n.d. |
| [¹²⁵I]I⁻ | 25.7±3.2 | 30.5±0.7 | 51.0±4.2 | 85.0±1.4 | 99.0±0.0 | n.d. |
| | | | | | | |
| Feces | 0-24 h | 24-48 h | | | | |
| Unchanged [¹²⁵I]**(1)** | 5.0±0.0 | 2.5±0.7 | | | | |
| Amine [¹²⁵I]I | 16.0±0.0 | 14.0±0.0 | | | | |
| Amine [¹²⁵I]II | 6.5±0.7 | 2.5±0.7 | | | | |
| Acid [¹²⁵I]I | 7.5±0.7 | 8.0±1.4 | | | | |
| Acid [¹²⁵I]II | 3.0±0.0 | 3.5±0.7 | | | | |
| [¹²⁵I]I⁻ | 8.0±0.0 | 27.0±1.4 | | | | |

| | | | | | | |
|---|---|---|---|---|---|---|
| *n.d.: not determined* | | | | | | |

### 10.4. Internal radiotherapy with compound [¹³¹I](1)

### 10.4.1 Experimental protocol.

Compound **(1)** was labelled with iodine-131, at high specific activity, from the corresponding stannane precursor, as described in detail in example 8. The labeling was performed with a radiochemical yield of 80% and a radiochemical purity> 99%. The antitumour activity of the compound [¹³¹I]**(1)** was evaluated in the melanoma model B16BL6, (primary melanoma model exhibiting spontaneous pulmonary dissemination) after two intravenous injections of 20 MBq, at day-7 and day-11 stages post inoculation. The antitumour activity of the compound [¹³¹I]**(1)** was also evaluated in SKMEL-3 human melanoma model after injections of 25 MBq of [¹³¹I]**(1)**: Two groups of mice were used receiving two injections at day-33 and day-40 or three injections of at day-33, day-40 and day-47 post inoculation. Results were compared to control group injected with saline.

### 10.4.2 Results

Targeted radionuclide therapy studies demonstrated that [¹³¹I]**(1)** was able to deliver a high radiation dose to melanoma tumours, inducing a significant reduction of tumoural growth rate (doubling time of 2.9±0.5 days *vs* 1.8±0.3 days in controls (*p*=*0.006*) for B16BL6 tumours and 26.5±7.8 days *vs* 11.0±3.8 days in controls (*p*<*0.0001*) for SKMEL-3 tumours). An increase in median survival time was observed in treated B16BL6 bearing-mice (27 days *vs* 21 days in controls *p*<0.0001) as well as in treated SKMEL-3 bearing-mice (111 days *vs* 74 days in controls; *p*<*0.0001*). Moreover, inhibition of metastasis dissemination could be demonstrated in B16BL6 melanoma model.

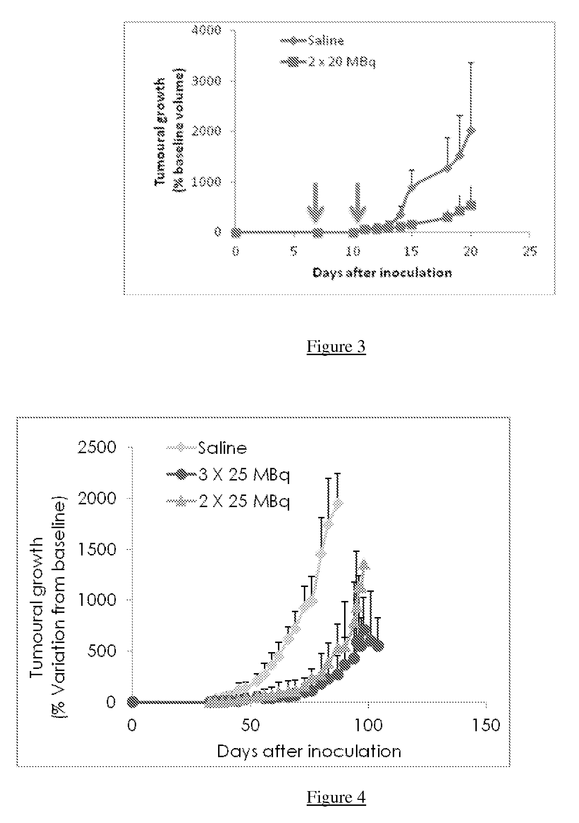

Figure 3. Effect of [¹³¹I]**(1)** on murine melanoma B16BL6 tumour growth. [¹³¹I]**(1)** was administered at 20 MBq at day-7 and day-11 after inoculation. Arrows indicate treatment days. Tumour growth data are expressed as the percentage change in tumour volume relative to baseline.

Figure 4. Effect of [¹³¹I]**(1)** on human melanoma SKMEL-3 tumour growth. *[¹³¹I]**(1)** was administered at 25 MBq once weekly for 2 or 3 weeks. Tumour growth data are expressed as the percentage change in tumour volume relative to baseline.*

### 10.5. Calculation of absorbed dose to the tumour and organs

### 10.5.1 Protocol

Dose calculations based on the Medical Internal Radiation Dose (MIRD) methodology were obtained from [¹²⁵I]**(1)** kinetic data on B16B16 melanoma-bearing C57BL/6J mice and were extrapolated to [¹³¹I]**(1)**. The absorbed dose was calculated for each organ using the cumulative activity, the equilibrium dose constant of iodine 131, the absorbed dose fractions calculated according to the nature and energy of its emissions, the geometric volumes of the organs and tumour and considering tissue densities equal to 1 g.cm⁻³.

### 10.5.2 Results

**Table 6: Projected organ delivered dose**

| **Organs** | **Dose for iodine-125 (Gy)** | **Dose for iodine 131 (Gy)** | | |
|---|---|---|---|---|
| | | **Effective half life (h)** | **Biological half life (h)** | **Delivered Dose (Gy) - 2 doses** |
| Blood | 0.16 | 32.02 | 27.45 | 1.18 |
| Tumour | 10.74 | 114.18 | 71.67 | 102.30 |
| Eyes | 51.55 | 356.77 | 125.03 | 83.03 |
| Liver | 0.67 | 29.89 | 25.87 | 4.92 |
| Kidney | 0.58 | 32.22 | 27.60 | 4.22 |
| Skin | 0.51 | 60.79 | 46.20 | 3.35 |
| Adipose | 0.10 | 47.78 | 38.28 | 0.67 |
| Muscle | 0.08 | 46.36 | 37.36 | 0.56 |
| bones | 0.07 | 13.85 | 12.92 | 0.53 |
| Stomach | 1.20 | 31.89 | 27.36 | 8.73 |
| Small Bowels | 0.64 | 26.99 | 23.67 | 4.74 |
| Colon with Faeces | 1.72 | 26.92 | 28.48 | 12.80 |
| Spleen | 0.96 | 33.43 | 33.66 | 6.98 |
| Pancreas | 0.26 | 40.79 | 29.04 | 1.80 |
| Lung | 0.33 | 34.21 | 37.73 | 2.36 |
| Heart | 0.27 | 46.93 | 68.51 | 1.84 |
| Brain | 0.03 | 41.63 | 27.55 | 0.25 |
| Testes | 0.10 | 32.16 | 27.57 | 0.73 |
| Bladder (empty) | 1.17 | 32.17 | 27.57 | 8.55 |

### 10.6. Assessment of compound (1) biodistribution by Secondary ion mass spectrometry analysis

### 10.6.1 Experimental protocol.

B16BL6 cells were injected intravenously into male C57BL/6J mice to obtain, within 12 days, tumour cell colonies in lungs mimicking pulmonary micrometastases. After i.v. administration of compound (1) (0.1 µmol/mice in 0.2 mL of a hydroalcoholic solution (5% v/v, 0.9% NaCl) per mouse), mice were killed 1 h post injection. Lungs were removed and small pieces of tissue including B16 melanoma colonies were isolated and fixed by slam freezing on an LN2-precooled metal mirror. Samples were dehydrated by freeze drying starting at -110 to -10 °C before embedding in Spurr resin. Serial sections of 0.4 µm thick were deposited on stainless steel holders for SIMS analysis or on glass slides for light microscopy observation.

### 10.6.2. Results of SIMS analysis

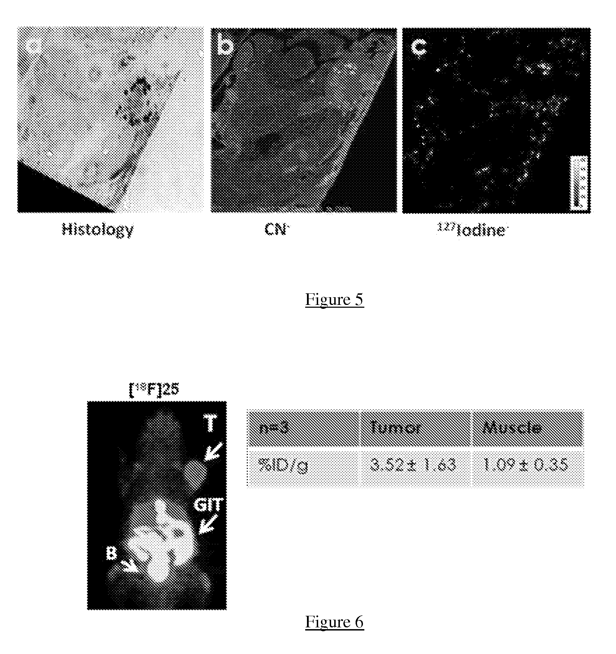

Figure 5: Secondary ion mass spectrometry (SIMS) images used to determine the distribution of compound **(1).** SIMS allows direct identification of chemical elements with high sensitivity and specificity and can be applied to visualize elemental distribution (chemical mapping). Images centered on one B16BL6 melanoma cells colony 1 h after compound **(1)** injection. Figures 5b and 5c showed distribution of CN- and Iodine-127 ions revealing various tissue structures in particular melanin polymers. Furthermore, the histological image (a) revealed melanin grains and clusters. Superimposition of the CN⁻ and iodide-127 signals was observed, indicating a perfect colocalisation of compound (1) with melanin polymers. These data showed the colocalisation of compound (1) with melanin.

Figure 5. SIMS images centered on one B16BL6 melanoma cells colony 1 h after compound **(1)** (0.1µmol/mice) injection. Histological image is shown in (a). CN⁻ (b) and ¹²⁷**I**⁻ ions (c).

### EXAMPLE 11: Comparative data with respect to compound 25 of WO2009/095872

### 11.1. Comparative issues on the radiosynthesis aspect

Synthesis of compound *N*-[2-[[*N*-ethyl-*N*-(2-fluoroethyl)]amino]ethyl]-6-iodoquinoxaline-2-carboxamide **(25)** was described in WO2009/095872 (example 4). However, no pharmacological data was disclosed in connection to it.

Radiofluorination of said comparative compound **(25)** may be performed according to the following two-step procedure, also described under typical example 3:
Radiosynthesis of compounds [¹⁸F]**(1)**, [¹⁸F]**(5)** and [¹⁸F]**(7)**, as described in detail in example 9, was compared to the synthesis of comparative compound [¹⁸F]**(25)**. It was stated that the radiosynthesis of compounds [¹⁸F]**(1)**, [¹⁸F]**(5)** and [¹⁸F]**(7)** are easier to perform and that the yields and preparation times are better.

| | **Preparation time** | **Radiochemical Yield (decay corrected)** | **Radiochemical Purity** |
|---|---|---|---|
| **[¹⁸F](25)** | 110-130 min (2-step process) | 6-10% | > 95% |
| **[¹⁸F](1)** | 57 min | 21% | > 99% |
| **[¹⁸F](5)** | 65 min | 34% | > 99% |
| **[¹⁸F](7)** | 85 min (2-step process) | 36% | > 99% |

### 11.2. Comparative data with respect to PET imaging

### 11.2.1 Protocol : see protocol of example 10

### 11.2.2 In vivo PET imaging

PET imaging was performed 1 h post injection of tracers in B16BL6 tumour bearing mice at day-15 after tumours cell inoculation (cf figures 1 and 6).
Comparison of PET imaging, shows a high and specific tumoural uptake of [¹⁸F]**1** and [¹⁸F]**7** as early as 1 h post injection (**14.33** ± **2**.**11** and **14**.**55**% ID/g, respectively), with a tumour/muscle ratio of **11.04** ± **2.87** and **17.41**, respectively. Comparatively, **[¹⁸F](5)** displays lower specific tumoural uptake (**6.63 ± 1.64%** ID/g) as well as tumour/muscle ratio **(6.62 ± 1.90).**

It comes out from the PET imaging that [¹⁸F](**1**) is particularly interesting due: i) to its tumoural tropism (**14.33 ± 2.11%** ID/g) and tumour to muscle ratio **(11.04 ± 2.87),** highly superior to the fluoroethyl analogue [¹⁸F]**(25)** at 1 h p.i., and ii) to its lower gastro intestinal tract signal.

Figure 6 displays a PET image of a B16B16 tumour bearing mice performed at day 15, 1 h after injection of [¹⁸F]**(25)**. Tumour (T), gastrointestinal tract (GIT), and bladder (B) are indicated.

The previous results show that the compounds of the present invention can be used for the diagnosis with SPECT or PET imaging and/or the treatment of melanoma by targeted radionuclide therapy.

According to one of its aspects, the present invention relates to the use of an effective amount of a compound of formulae (I), (Ia) or (Ib) or one of its pharmaceutically acceptable salts for the preparation of a composition for the diagnosis and/or the treatment of melanoma.

According to another aspect, the present invention relates to compounds of formulae (I), (Ia) or (Ib) or one of its pharmaceutically acceptable salts as radiopharmaceutical and/or imaging agent for the diagnosis and/or the treatment of melanoma.

Described herein is a method for the diagnosis and/or the treatment of melanoma wherein it comprises at least one step of administration to a patient suffering from melanoma or potentially suffering from melanoma of an effective amount of a coumpound of formulae (I), (Ia) or (Ib) or one of its pharmaceutically acceptable salts, said coumpound being beforehand radiolabelled.

According to another one of its aspects, the present invention relates to a radiopharmaceutical composition comprising a radiolabelled compound of formulae (I), (Ia) or (Ib) or anyone of compound **(1)** to **(8)** or of its pharmaceutically acceptable salt. The radio labelling occurs in particular by means of a radionuclide as described above.

According to another one of its aspects, the present invention relates to a radiopharmaceutical composition comprising, as active principle, a compound of formulae (I), (Ia) or (Ib) according to the invention, wherein it comprises a fluorine-18 atom or one of its pharmaceutically acceptable salts.

The said radiopharmaceutical composition advantageously comprises an effective amount of such a compound of formulae (I), (Ia) or (Ib), wherein it comprises a fluorine-18 atom, and also one or more excipients. Such excipients are chosen according to the type of formulation.

The present invention furthermore relates to compound of formulae (I), (Ia) or (Ib) or one of its pharmaceutically acceptable salts for use for the preparation of a radiopharmaceutical composition intended for medical imaging, more particularly for the diagnosis of melanoma.

According to another aspect, the present invention relates to compounds of formulae (I), (Ia) or (Ib) or one of its pharmaceutically acceptable salts for use as medical imaging agents, more particularly for the diagnosis of melanoma.

It is also described a method of PET imaging which employs a fluorine-18 radiolabelled compound of formulae (I), (Ia) or (Ib) as described above.

The patient/subject may be an animal, mammal or human.

It is also described a method of imaging comprising the following steps:
- introducing the radiolabelled compound of formulae (I), (Ia) or (Ib) into a subject,
- imaging a part or the whole body of the subject.

It is also described a noninvasive method for the determination of the tissue distribution of tumour cells of melanoma on the human body, comprising the stages of at least one injection of a radiopharmaceutical composition comprising at least one compound of formulae (I), (Ia) or (Ib), wherein it comprises a fluorine-18 atom, or one of its pharmaceutically acceptable salts and of at least one determination of the concentration of the radioactivity.

Methods of PET imaging are well known to the man skilled in the art.

Likewise, the present invention relates to a radiolabelled compound of formulae (I), (Ia) or (Ib) or one of its pharmaceutically acceptable salts or a radiolabelled compound of formula (1) to (8) as defined above for use for medical imaging, more particularly for the diagnosis or melanoma and/or for the treatment of melanoma.

According to another aspect, the present invention relates to compounds of formulae (I), (Ia) or (Ib) or one of its pharmaceutically acceptable salts for use as radiopharmaceutical compounds for the treatment of melanoma.

It is also described a method for the treatment of melanoma which comprises the administration to a patient suffering from melanoma and more particularly from disseminated melanoma of an effective amount of a compound of formulae (I), (Ia) or (Ib), wherein it comprises a iodine-131 atom, or one of its pharmaceutically acceptable salts.

Another aspect of the invention pertains to unlabelled compound of formulae (I), (Ia) or (Ib) as described above and an alkaline radioactive halide for use in the manufacture of a medicament for the use in the diagnosis and/or the treatment of melanoma.

According to another aspect, the present invention relates to unlabelled compounds of formulae (I), (Ia) or (Ib) or one of its pharmaceutically acceptable salts for use, when combined with an alkaline radioactive halide, as radiopharmaceutical and/or imaging agent for the diagnosis and/or the treatment of melanoma.

It is also described a method of treatment of melanoma comprising a step of imaging with a radiolabelled compound of formulae (I), (Ia) or (Ib) by fluorine-18 PET for diagnosis of melanoma lesions allowing, in a subject, a staging of the disease and then by treating said melanoma lesions, for example by injecting a compound of formulae (I), (Ia) or (Ib) which can be radiolabelled by iodine-131, astatine-211 or iodine-125, in particular denoting the same chemical structure with the exception of labelling, and following the evolution of the melanoma and the efficacy of the treatment by one or more successive imaging steps by fluorine-18 PET imaging. Indeed the fluorine-18 PET imaging allows a quantization of radioactivity present in the tumor, which is of particular interest to dosimetry studies in order to evaluate and adjust the therapeutic treatment of melanoma.

According to a particular aspect, the chemical structure for the imaging and the treatment is identical, except with respect to labelling, which is carried out on demand for each specific application.

The radiolabelled compound according to the present invention may be administred to a subject/patient by any convenient route of administration, whether systemically/peripherally or topically.

## Claims

1. Compound of formula (I): 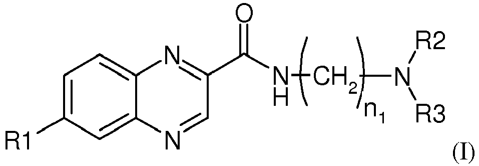 in which
R1 represents Sn(R)₃, B(OH)₂, B(OR)₂, a halogen atom, NO₂, a radionuclide or a -N⁺(R)₃ group, where R is a (C₁-C₆) alkyl group,
n₁ is an integer varying from 1 to 4,
R2 represents a hydrogen atom or a (C₁-C₆)alkyl group,
R3 represents:
- a -(CH₂CH₂O)ₙ₂-(CH₂)ₙ₃-X group, where n₂ is an integer varying from 1 to 12 and n₃ is an integer varying from 1 to 4, or
- a -(CH₂CH₂O)ₙ₄-(CH₂)ₙ₅-Y group with n₄ representing an integer varying from 0 to 12, n₅ representing an integer from 1 to 4, and Y representing a -C=C-H, a -N₃ or a -Ar-(CH₂)ₙ₆-(OCH₂CH₂)ₙ₇-X group with
Ar being 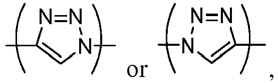 n₆ representing an integer varying from 1 to 4 and n₇ an integer varying from 0 to 12, and
X represents a halogen atom, a radionuclide or a -OSO₂R' group, where R' is a CF₃, CH₃, *t*-Bu, Ph, *p*-NO₂Ph, *p*-BrPh or *p*-CH₃Ph group,
and their addition salts with pharmaceutically acceptable acids.

2. Compound of formula (I) according to claim 1, wherein R2 is an ethyl group.

3. Compound of formula (I) according to claim 1 or 2, wherein n₁ is 2.

4. Compound of formula (I) according to anyone of claim 1 to 3, wherein R1 is a iodine atom, a radionuclide or Sn(R)₃, where R is a (C₁-C₆) alkyl group, and more particularly R1 is a iodine atom.

5. Compound of formula (I) according to anyone of claims 1 to 4, wherein R3 represents:
- a -(CH₂CH₂O)ₙ₂-(CH₂)₂-X group, where n₂ is an integer varying from 3 to 7,
- a -CH₂-Y group with Y representing a -C=C-H, a -N₃ or a
- Ar-(CH₂)ₙ₆-X group with
Ar being 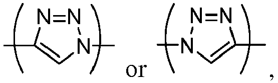 n₆ representing an integer varying from 1 to 4, and
X is a halogen atom, a radionuclide or a -OSO₂R' group, where R' is a CF₃, CH₃, *t*-Bu, Ph, *p*-NO₂Ph, *p*-BrPh or *p*-CH₃Ph group.

6. Compound of formula (Ia) according to claim 1 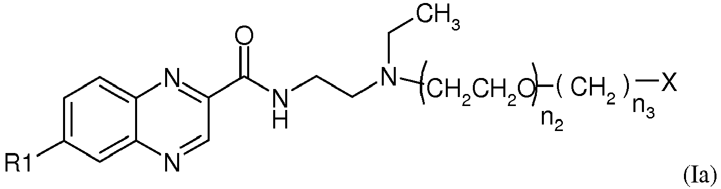 in which
R1 represents Sn(R)₃, B(OH)₂, B(OR)₂, a halogen atom, NO₂, a radionuclide or a -N⁺(R)₃ group, where R is a (C₁-C₆)alkyl group,
n₂ is an integer varying from 1 to 12,
n₃ is an integer varying from 1 to 4, and
X is a halogen atom, a radionuclide or a -OSO₂R' group, where R' is a CF₃, CH₃, *t*-Bu, Ph, *p*-NO₂Ph, *p*-BrPh or *p*-CH₃Ph group,
and their addition salts with pharmaceutically acceptable acids.

7. Compound of formula (Ia) according to claim 6, wherein n₂ varies between 3 and 7 and n₃ is 2.

8. Compound of formula (Ia) according to claim 6 or 7, wherein R1 represents a iodine atom, a radionuclide or Sn(R)₃, where R is a (C₁-C₆)alkyl group, and more particularly R1 represents a iodine atom or an optionally labelled iodine atom, and X represents an optionally labelled fluorine atom or a -OSO₂R' group, where R' is a CF₃, a *t-*Bu or a CH₃ group, and more particularly a CH₃ group.

9. Compound of formula (Ib) according to claim 1 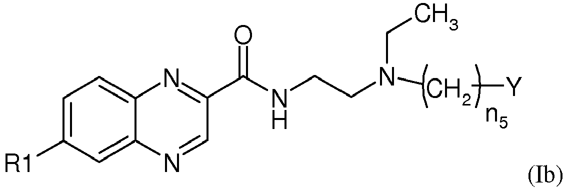 in which
R1 represents Sn(R)₃, B(OH)₂, B(OR)₂, a halogen atom, NO₂, a radionuclide or a -N⁺(R)₃ group, where R is a (C₁-C₆)alkyl group,
n₅ represents an integer varying from 1 to 4, and
Y represents a -C=C-H, a -N₃ or a -Ar-(CH₂)ₙ₆-(OCH₂CH₂)ₙ₇-X group with
Ar being 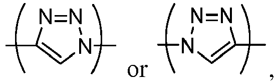 n₆ representing an integer varying from 1 to 4, n₇ an integer varying from 0 to 12 and X a halogen, a radionuclide or a -OSO₂R' group, where R' is a CF₃, CH₃, *t*-Bu, Ph, *p*-NO₂Ph, *p*-BrPh or *p*-CH₃Ph,
and their addition salts with pharmaceutically acceptable acids.

10. Compound of formula (Ib) according to claim 9, wherein n₅ is 1 and Y represents a -C=C-H, a -N₃ or a -Ar-(CH₂)ₙ₆-X group with
Ar being 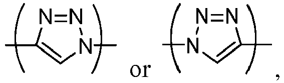 n₆ representing an integer varying from 1 to 4 and X a halogen, a radionuclide or a OSO₂R' group, where R' is a CF₃, CH₃, *t*-Bu, Ph, *p*-NO₂Ph, *p*-BrPh or *p*-CH₃Ph.

11. Compound of formula (Ib) according to claim 9 or 10, wherein R1 represents a iodine atom, a radionuclide or Sn(R)₃, where R is a (C₁-C₆)alkyl group, and more particularly R1 represents a iodine atom, and Y represents a -C=C-H or a -Ar-(CH₂)ₙ₆-X group with
Ar being 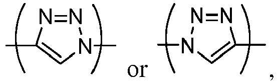 n₆ representing an integer varying from 1 to 4 and X representing an optionally labelled fluorine atom or a -OSO₂R' group, where R' is a CF₃ or a CH₃ group, and more particularly a CH₃ group.

12. A radiolabelled compound according to any one of the preceding claims, wherein said radiolabelling occurs by means of a radionuclide which is a radioisotope chosen from iodine-123, iodine-124, iodine-125, iodine-131, bromine-75, bromine-76, bromine-77, fluorine-18, astatine-210 or astatine-211, and particularly is chosen among fluorine-18, iodine-123, iodine-124, iodine-125 and iodine-131.

13. Compound according to anyone of the preceding claims, chosen from:
- *N*-(12-ethyl-1-fluoro-3,6,9-trioxa-12-azatetradecan-14-yl)-6-iodoquinoxaline-2-carboxamide **(1),**
- *N*-(12-ethyl-1-methanesulfonyloxy-3,6,9-trioxa-12-azatetradecan-14-yl)-6-iodoquinoxaline-2-carboxamide **(2)**,
- *N*-(12-ethyl-1-fluoro-3,6,9-trioxa-12-azatetradecan-14-yl)-6-(tributylstannyl)quinoxaline-2-carboxamide **(4),**
- *N*-(24-ethyl-1-fluoro-3,6,9,12,15,18,21-heptaoxa-24-azahexacosan-26-yl)-6-iodoquinoxaline-2-carboxamide **(5),**
- *N*-(24-ethyl-1-methanesulfonyloxy-3,6,9,12,15,18,21-heptaoxa-24-azahexacosan-26-yl)-6-iodoquinoxaline-2-carboxamide **(6),**
- *N*-[2-[(*N*-ethyl)-[[1-(2-fluoroethyl)-1*H*-(1,2,3)triazol-4-yl]methyl]amino]ethyl]-6-iodoquinoxaline-2-carboxamide **(7),**
- *N*-[2-[(*N*-ethyl)-(*N*-prop-2-ynyl)amino]ethyl]-6-iodoquinoxaline-2-carboxamide **(8),**
and their pharmaceutically acceptable salts and more particularly their hydrochlorides or bases.

14. Radiopharmaceutical composition comprising, as active principle, a radiolabelled compound of formula (I), (Ia) or (Ib) or one of its pharmaceutically acceptable salts as defined according to any one of claims 1 to 12 or one of compound **(1), (2), (4), (5), (6), (7)** and **(8)** as defined in claim 13.

15. A radiolabelled compound of formula (I), (Ia) or (Ib) as defined according to anyone of claims 1 to 12 or of one of its pharmaceutically acceptable salts or a radiolabelled compound of formula or one of compound **(1), (2), (4), (5), (6), (7)** and **(8)** as defined in claim 13, for use in the medical imaging, and more particularly for use in the diagnosis of melanoma and/or for use in the treatment of melanoma.

## Patentansprüche

1. Verbindung der Formel (I): 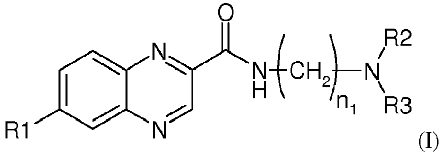 in der
R1 SN(R)₃, B(OH)₂, B(OR)₂, ein Halogenatom, NO₂, ein Radionuklid oder eine -N⁺(R)₃ Gruppe darstellt, wobei R eine (C₁-C₆) Alkylgruppe ist,
n₁ eine ganze Zahl variierend von 1 bis 4 ist,
R2 ein Wasserstoffatom oder eine (C₁-C₆) Alkylgruppe darstellt,
R3:
- eine -(CH₂CH₂O)ₙ₂-(CH₂)ₙ₃-X Gruppe, wobei n₂ eine ganze Zahl variierend von 1 bis 12 ist und n₃ eine ganze Zahl variierend von 1 bis 4 ist, oder
- eine -(CH₂CH₂O)ₙ₄-(CH₂)ₙ₅-Y Gruppe darstellt, wobei n₄ eine ganze Zahl variierend von 0 bis 12 darstellt, n₅ eine ganze Zahl variierend von 1 bis 4 darstellt und Y eine -C≡C-H, eine -N₃ oder eine -Ar-(CH₂)ₙ₆-(OCH₂CH₂)ₙ₇-X Gruppe darstellt, wobei Ar 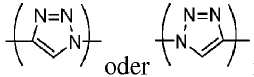 ist, n₆ eine ganze Zahl variierend von 1 bis 4 darstellt und n₇ eine ganze Zahl von 0 bis 12 darstellt, und
X ein Halogenatom, ein Radionuklid oder eine -OSO₂R' Gruppe darstellt, wobei R' eine CF₃, CH₃, *t*-Bu, Ph, *p*-NO₂Ph, *p*-BrPh oder *p*-CH₃Ph Gruppe ist,
und ihre Additionssalze mit pharmazeutisch annehmbaren Säuren.

2. Verbindung der Formel (I) gemäß Anspruch 1, wobei R2 eine Ethylgruppe ist.

3. Verbindung der Formel (I) gemäß Anspruch 1 oder 2, wobei n₁ 2 ist.

4. Verbindung der Formel (I) gemäß einem der Ansprüche 1 bis 3, wobei R1 ein IodAtom, ein Radionuklid oder SN(R)₃ ist, wobei R eine (C₁-C₆) Alkylgruppe ist, und insbesondere ist R1 ein Iod-Atom.

5. Verbindung der Formel (I) gemäß einem der Ansprüche 1 bis 4, wobei R3:
- eine -(CH₂CH₂O)ₙ₂-(CH₂)₂-X Gruppe, wobei n₂ eine ganze Zahl variierend von 3 bis 7 ist,
- eine -CH₂-Y Gruppe darstellt, wobei Y eine -C≡C-H, eine -N₃ oder eine -Ar-(CH₂)ₙ₆-X Gruppe darstellt, wobei
Ar 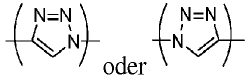 ist, n₆ eine ganze Zahl variierend von 1 bis 4 darstellt, und
X ein Halogenatom, ein Radionuklid oder eine -OSO₂R' Gruppe ist, wobei R' eine CF₃, CH₃, *t*-Bu, Ph, *p*-NO₂Ph, *p*-BrPh oder *p*-CH₃Ph Gruppe ist.

6. Verbindung der Formel (Ia) gemäß Anspruch 1 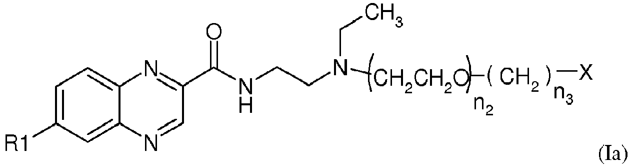 in der
R1 SN(R)₃, B(OH)₂, B(OR)₂, ein Halogenatom, NO₂, ein Radionuklid oder eine -N⁺(R)₃ Gruppe darstellt, wobei R eine (C₁-C₆) Alkylgruppe ist,
n₂ eine ganze Zahl variierend von 1 bis 12 ist,
n₃ eine ganze Zahl variierend von 1 bis 4 ist und
X ein Halogenatom, ein Radionuklid oder eine -OSO₂R' Gruppe ist, wobei R' eine CF₃, CH₃, *t*-Bu, Ph, *p*-NO₂Ph, *p*-BrPh oder *p*-CH₃Ph Gruppe ist,
und ihre Additionssalze mit pharmazeutisch annehmbaren Säuren.

7. Verbindung der Formel (Ia) gemäß Anspruch 6, wobei n₂ zwischen 3 und 7 variiert und n₃ 2 ist.

8. Verbindung der Formel (Ia) gemäß Anspruch 6 oder 7, wobei R1 ein Iod-Atom, ein Radionuklid oder SN(R)₃ darstellt, wobei R eine (C₁-C₆) Alkylgruppe ist, und insbesondere stellt R1 ein Iod-Atom oder ein optional markiertes Iod-Atom dar, und X ein optional markiertes Fluor-Atom oder eine -OSO₂R' Gruppe darstellt, wobei R' eine CF₃, eine *t*-Bu oder eine CH₃ Gruppe ist und insbesondere eine CH₃ Gruppe.

9. Verbindung der Formel (Ib) gemäß Anspruch 1 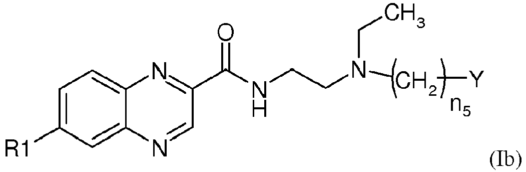 in der
R1 SN(R)₃, B(OH)₂, B(OR)₂, ein Halogenatom, NO₂, ein Radionuklid oder eine -N⁺(R)₃ Gruppe darstellt, wobei R eine (C₁-C₆) Alkylgruppe ist,
n₅ eine ganze Zahl variierend von 1 bis 4 darstellt und
Y eine -C≡C-H, eine -N₃ oder eine -Ar-(CH₂)ₙ₇-X Gruppe darstellt, wobei Ar 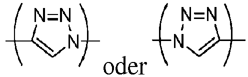 ist, n₆ eine ganze Zahl variierend von 1 bis 4 darstellt, n₇ eine ganze Zahl variierend von 0 bis 12 darstellt und X ein Halogenatom, ein Radionuklid oder eine -OSO₂R' Gruppe darstellt, wobei R' ein CF₃, CH₃, *t*-Bu, Ph, *p*-NO₂Ph, *p*-BrPh oder *p*-CH₃Ph ist,
und ihre Additionssalze mit pharmazeutisch annehmbaren Säuren.

10. Verbindung der Formel (Ib) gemäß Anspruch 9, wobei n₅ 1 ist und Y eine -C≡C-H, eine -N₃ oder eine -Ar-(CH₂)ₙ₆-X Gruppe darstellt, wobei Ar 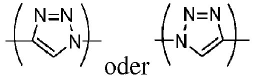 ist, n₆ eine ganze Zahl variierend von 1 bis 4 darstellt und X ein Halogenatom, ein Radionuklid oder eine -OSO₂R' Gruppe darstellt, wobei R' ein CF₃, CH₃, *t*-Bu, Ph, *p*-NO₂Ph, *p*-BrPh oder *p*-CH₃Ph ist.

11. Verbindung der Formel (Ib) gemäß Anspruch 9 oder 10, wobei R1 ein Iod-Atom, ein Radionuklid oder SN(R)₃ darstellt, wobei R eine (C₁-C₆) Alkylgruppe ist, und insbesondere stellt R1 ein Iod-Atom dar, und Y eine -C≡C-H oder eine -Ar-(CH₂)ₙ₆-X Gruppe darstellt, wobei
Ar 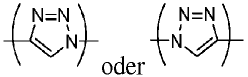 ist, n₆ eine ganze Zahl variierend von 1 bis 4 darstellt und X ein optional markiertes Fluor-Atom oder eine -OSO₂R' Gruppe darstellt, wobei R' eine CF₃ oder eine CH₃ Gruppe ist, und insbesondere eine CH₃ Gruppe.

12. Eine radioaktiv markierte Verbindung gemäß einem der voranstehenden Ansprüche, wobei das radioaktive Markieren mittels eines Radionuklids erfolgt, welches ein Radioisotop ausgewählt aus Iod-123, Iod-124, Iod-125, Iod-131, Brom-75, Brom-76, Brom-77, Fluor-18, Astatin-210 oder Astatin-211 ist und insbesondere ausgewählt ist aus Fluor-18, Iod-123, Iod-124, Iod-125 und Iod-131.

13. Verbindung gemäß einem der voranstehenden Ansprüche, ausgewählt aus:
- N-(12-Ethyl-1-fluoro-3,6,9-trioxa-12-azatetradecan-14-yl)-6-iodochinoxalin-2-carboxamid **(1),**
- N-(12-Ethyl-1-methansulfonyloxy-3,6,9-trioxa-12-azatetradecan-14-yl)-6-iodochinoxalin-2-carboxamid **(2),**
- N-(12-Ethyl-1-fluoro-3,6,9-trioxa-12-azatetradecan-14-yl)-6-(tributylstannyl)chinoxalin-2-carboxamid **(4),**
- N-(24-Ethyl-1-fluoro-3,6,9,12,15,18,21-heptaoxa-24-azahexacosan-26-yl)-6-iodochinoxalin-2-carboxamid **(5),**
- N-(24-Ethyl-1-methansulfonyloxy-3,6,9,12,15,18,21-heptaoxa-24-azahexacosan-26-yl)-6-iodochinoxalin-2-carboxamid **(6),**
- N-[2-[(N-Ethyl)-[[1-(2-fluoroethyl)-1H-(1,2,3)triazol-4-yl]methyl]amino]ethyl]-6-iodochinoxalin-2-carboxamid **(7),**
- N-[2-[(N-Ethyl)-(N-prop-2-inyl)amino]ethyl]-6-iodochinoxalin-2-carboxamid **(8),**
und ihre pharmazeutisch annehmbaren Salze und insbesondere ihre Hydrochloride oder Basen.

14. Radiopharmazeutische Zusammensetzung umfassend, als Wirkstoff, eine radioaktiv markierte Verbindung der Formel (I), (Ia) oder (Ib) oder eines ihrer pharmazeutisch annehmbaren Salze wie gemäß einem der Ansprüche 1 bis 12 definiert oder eine von Verbindung **(1), (2), (4), (5), (6), (7)** und **(8)** wie in Anspruch 13 definiert.

15. Eine radioaktiv markierte Verbindung der Formel (I), (Ia) oder (Ib) wie gemäß einem der Ansprüche 1 bis 12 definiert oder eines ihrer pharmazeutisch annehmbaren Salze oder eine radioaktiv markierte Verbindung der Formel oder eine von Verbindung **(1), (2), (4), (5), (6), (7)** und **(8)** wie in Anspruch 13 definiert, zur Verwendung in der medizinischen Bildgebung und insbesondere zur Verwendung in der Diagnose eines Melanoms und/oder zur Verwendung in der Behandlung eines Melanoms.

## Revendications

1. Composé de formule (I) : 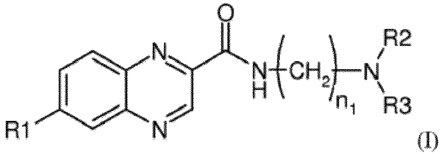 dans laquelle
R1 représente Sn(R)₃, B(OH)₂, B(OR)₂, un atome d'halogène, NO₂, un radionucléide ou un groupe -N⁺(R)₃, où R représente un groupe alkyle en C₁ à C₆,
n₁ est un nombre entier variant de 1 à 4,
R2 représente un atome d'hydrogène ou un groupe alkyle en C₁ à C₆,
R3 représente :
- un groupe -(CH₂CH₂O)ₙ₂-(CH₂)ₙ₃-X, où n₂ est un nombre entier variant de 1 à 12 et n₃ est un nombre entier variant de 1 à 4, ou
- un groupe -(CH₂CH₂O)ₙ₄-(CH₂)ₙ₅-Y, où n₄ est un nombre entier variant de 0 à 12 et n₅ est un nombre entier variant de 1 à 4, et Y représente un groupe -C≡C-H, -N₃ ou -Ar-(CH₂)ₙ₆-(OCH₂CH₂)ₙ₇-X où
Ar représente 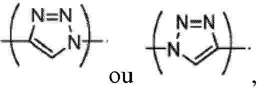 n₆ est un nombre entier variant de 1 à 4 et n₇ est un nombre entier variant de 0 à 12, et
X représente un atome d'halogène, un radionucléide ou un groupe -OSO₂R', où R' représente un groupe CF₃, CH₃, *t*-Bu, Ph, *p*-NO₂Ph, *p*-BrPh ou *p*-CH₃Ph,
et ses sels d'addition avec des acides pharmaceutiquement acceptables.

2. Composé de formule (I) selon la revendication 1, dans lequel R2 est un groupe éthyle.

3. Composé de formule (I) selon la revendication 1 ou 2, dans lequel n₁ est 2.

4. Composé de formule (I) selon l'une quelconque des revendications 1 à 3, dans lequel R1 représente un atome d'iode, un radionucléide ou Sn(R)₃, où R représente un groupe alkyle en C₁ à C₆, et plus particulièrement R1 représente un atome d'iode.

5. Composé de formule (I) selon l'une quelconque des revendications 1 à 4, dans lequel R3 représente :
- un groupe -(CH₂CH₂O)ₙ₂-(CH₂)₂-X, où n₂ est un nombre entier variant de 3 à 7,
- un groupe -CH₂-Y où Y représente un groupe -C≡C-H, -N₃ ou -Ar-(CH₂)ₙ₆-X où
Ar représente 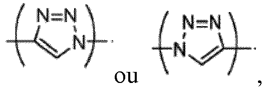 n₆ est un nombre entier variant de 1 à 4, et
X représente un atome d'halogène, un radionucléide ou un groupe -OSO₂R', où R' représente un groupe CF₃, CH₃, *t*-Bu, Ph, *p*-NO₂Ph, *p*-BrPh ou *p*-CH₃Ph.

6. Composé de formule (Ia) selon la revendication 1, 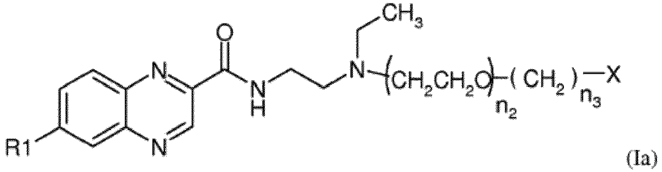 dans laquelle
R1 représente Sn(R)₃, B(OH)₂, B(OR)₂, un atome d'halogène, NO₂, un radionucléide ou un groupe -N⁺(R)₃, où R représente un groupe alkyle en C₁ à C₆,
n₂ est un nombre entier variant de 1 à 12,
n₃ est un nombre entier variant de 1 à 4, et
X représente un atome d'halogène, un radionucléide ou un groupe -OSO₂R', où R' représente un groupe CF₃, CH₃, *t*-Bu, Ph, *p*-NO₂Ph, *p*-BrPh ou *p*-CH₃Ph,
et ses sels d'addition avec des acides pharmaceutiquement acceptables.

7. Composé de formule (Ia) selon la revendication 6, dans lequel n₂ varie entre 3 et 7 et n₃ est 2.

8. Composé de formule (Ia) selon la revendication 6 ou 7, dans lequel R1 représente un atome d'iode, un radionucléide ou Sn(R)₃, où R représente un groupe alkyle en C₁ à C₆, et plus particulièrement R1 représente un atome d'iode ou un atome d'iode facultativement marqué, et X représente un atome de fluor facultativement marqué ou un groupe -OSO₂R', où R' représente un groupe CF₃, *t*-Bu ou CH₃, et plus particulièrement un groupe CH₃.

9. Composé de formule (Ib) selon la revendication 1, 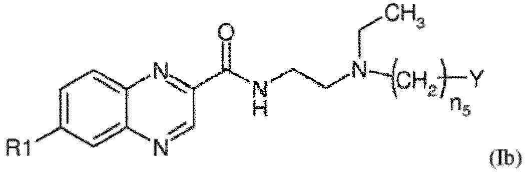 dans laquelle
R1 représente Sn(R)₃, B(OH)₂, B(OR)₂, un atome d'halogène, NO₂, un radionucléide ou un groupe -N⁺(R)₃, où R représente un groupe alkyle en C₁ à C₆,
n₅ est un nombre entier variant de 1 à 4,
Y représente un groupe -C≡C-H, -N₃ ou -Ar-(CH₂)ₙ₆-(OCH₂CH₂)ₙ₇-X où
Ar représente 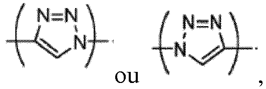 n₆ est un nombre entier variant de 1 à 4, n₇ est un nombre entier variant de 0 à 12, et X représente un atome d'halogène, un radionucléide ou un groupe -OSO₂R', où R' représente un groupe CF₃, CH₃, *t*-Bu, Ph, *p*-NO₂Ph, *p*-BrPh ou *p-*CH₃Ph,
et ses sels d'addition avec des acides pharmaceutiquement acceptables.

10. Composé de formule (Ib) selon la revendication 9, dans lequel n₅ est 1 et Y représente un groupe -C≡C-H, -N₃ ou -Ar-(CH₂)ₙ₆-X où
Ar représente 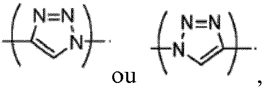 n₆ est un nombre entier variant de 1 à 4, et X représente un atome d'halogène, un radionucléide ou un groupe -OSO₂R', où R' représente un groupe CF₃, CH₃, *t*-Bu, Ph, *p*-NO₂Ph, *p*-BrPh ou *p*-CH₃Ph.

11. Composé de formule (Ib) selon la revendication 9 ou 10, dans lequel R1 représente un atome d'iode, un radionucléide ou Sn(R)₃, où R représente un groupe alkyle en C₁ à C₆, et plus particulièrement R1 représente un atome d'iode, et Y représente un groupe - C≡C-H, ou -Ar-(CH₂)ₙ₆-X où
Ar représente 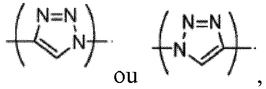 n₆ est un nombre entier variant de 1 à 4 et X représente un atome de fluor facultativement marqué ou un groupe -OSO₂R', où R' représente un groupe CF₃, *t*-Bu ou CH₃, et plus particulièrement un groupe CH₃.

12. Composé radiomarqué selon l'une quelconque des revendications précédentes, dans lequel ledit radiomarquage se produit par le biais d'un radionucléide qui est un radioisotope choisi parmi l'iode 123, l'iode 124, l'iode 125, l'iode 131, le brome 75, le brome 76, le brome 77, le fluor 18, l'astatine 210 ou l'astatine 211, et en particulier choisi parmi le fluor 18, l'iode 123, l'iode 124, l'iode 125 et l'iode 131.

13. Composé selon l'une quelconque des revendications précédentes, choisi parmi :
- N-(12-éthyl-1-fluoro-3,6,9-trioxa-12-azatétradécan-14-yl)-6-iodoquinoxaline-2-carboxamide (1),
- N-(12-éthyl-1-méthanesulfonyloxy-3,6,9-trioxa-12-azatétradécan-14-yl)-6-iodoquinoxaline-2-carboxamide (2),
- N-(12-éthyl-1-fluoro-3,6,9-trioxa-12-azatétradécan-14-yl)-6-(tributylstannyl)-quinoxaline-2-carboxamide (4),
- N-(24-éthyl-1-fluoro-3,6,9,12,15,18,21-heptaoxa-24-azahexacosan-26-yl)-6-iodoquinoxaline-2-carboxamide (5),
- N-(24-éthyl-1-méthanesulfonyloxy-3,6,9,12,15,18,21-heptaoxa-24-azahexacosan-26-yl)-6-iodoquinoxaline-2-carboxamide (6),
- N-[2-[(N-éthyl)-[[1-(2-fluoroéthyl)-1H-(1,2,3)triazol-4-yl]méthyl]amino]éthyl]-6-iodoquinoxaline-2-carboxamide (7),
- N-[2-[(N-éthyl)-(N-prop-2-ynyl)amino]éthyl]-6-iodoquinoxaline-2-carboxamide (8),
et leurs sels pharmaceutiquement acceptables et plus particulièrement leurs chlorhydrates ou leurs bases.

14. Composition radiomarquée comprenant, comme principe actif, un composé radiomarqué de formule (I), (Ia) ou (Ib) ou un de ses sels pharmaceutiquement acceptables tels que défini selon l'une quelconque des revendications 1 à 12 ou un composé (1), (2), (4), (5), (6), (7) ou (8) tel que défini selon la revendication 13.

15. Composé radiomarqué de formule (I), (Ia) ou (Ib) tel que défini selon l'une quelconque des revendications 1 à 12 ou d'un de ses sels pharmaceutiquement acceptables ou composé radiomarqué de formule ou composé (1), (2), (4), (5), (6), (7) ou (8) tel que défini selon la revendication 13, pour une utilisation dans l'imagerie médicale, et plus particulièrement pour une utilisation dans le diagnostic d'un mélanome et/ou pour une utilisation dans le traitement d'un mélanome.
